# EUROPEAN PATENT APPLICATION

(11) **EP 1 886 990 A1**
(43) Date of publication of application: **13.02.2008**
(21) Application number: 06380222.7
(22) Date of filing: 01.08.2006
(51) Int. Cl.: C07C 69/92, C07C 43/215, C07C 65/28, C07C 229/60, C07C 235/46, C07C 233/54, A61K 31/235, A61P 25/28

(54) **Phenyl-prenyl-ether derivatives for the treatment of cognitive neurodegenerative or neuronal diseases or disorders**

(71) Applicant: Neuropharma S.A., 28760 Tres Cantos (Madrid) (ES)
(72) Inventor: The designation of the inventor has not yet been filed
(74) Representative: ABG Patentes, S.L.

(57) **Abstract**

The present invention is related to a family of phenyl-prenyl-ether derivatives of formula (I), and to their use in the treatment of cognitive, neurodegenerative or neuronal diseases or disorders, such as Alzheimer's disease or Parkinson's Disease. The present invention also relates to pharmaceutical compositions comprising the same. Further, the present invention is directed to the use of the compounds in the manufacture of a medicament for the treatment and/or prevention of a cognitive, neurodegenerative or neuronal disease or disorder.

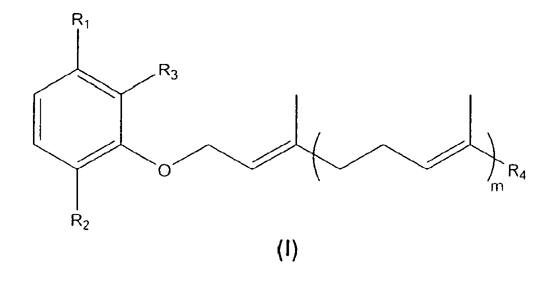

## Description

### FIELD OF THE INVENTION

The present invention is related to a family of phenyl-prenyl-ether derivatives of formula (I), and to their use in the treatment of cognitive, neurodegenerative or neuronal diseases or disorders, such as Alzheimer's disease or Parkinson's Disease. The present invention also relates to pharmaceutical compositions comprising the same. Further, the present invention is directed to the use of the compounds in the manufacture of a medicament for the treatment and/or prevention of a cognitive, neurodegenerative or neuronal disease or disorder.

### BACKGROUND OF THE INVENTION

Glycogen synthase kinase 3 (GSK-3) is a serine-threonine protein kinase comprised of α and β isoforms which phosphorylates diverse target proteins, such as enzymes or transcription factors. GSK-3β plays an important regulatory role in several signaling pathways of cellular processes, such as initiation of protein synthesis, cell proliferation, apoptosis or embryonic development (Discovery and development of GSK3 inhibitors for the treatment of type 2 diabetes, Wagman et al., Curr. Pharm. Des. 2004;10(10):1105-37). Disorders in many of these regulatory pathways are involved in human diseases, such as Parkinson's Disease (GSK-3beta inhibition/beta-catenin stabilization in ventral midbrain precursors increases differentiation into dopamine neurons, Castelo-Branco et al., J Cell Sci. 2004 Nov 15;117(Pt 24):5731-7), Alzheimer's Disease, type II diabetes, bipolar disorders, diseases caused by unicellular parasites that express GSK3 homologues (Pharmacological inhibitors of glycogen synthase kinases 3, Maijer L et al., Trends Pharmacol. Sci. 2004;25(9):471-80*)*) or prion-induced neurodegeneration (Prion peptide induces neuronal cell death through a pathway involving glycogen synthase kinase 3, Perez M. et al., Biochem. J. 2003; 372(Pt 1): 129-36).

An important regulatory process wherein GSK-3 takes part is the Wnt pathway. The Wnts are a family of cysteine-rich and glycosylated proteins which act as activators of different processes, such as cell growth differentiation, migration and fate (The Wnts, Miller JR, Genome Biol. 2002;3(1):REVIEWS3001). A key protein of this pathway is the β-catenin, which translocates to the nucleus and activates different genes when a Wnt binds to its receptor. A multi protein complex which includes APC (adenomatous polyposis coli) and axin, among other proteins, facilitates that GSK-3 phosphorilates β-catenin in several sites of its N-terminal domain. This event triggers the binding of ubiquitin to the phosphorylated β-catenin and its subsequent degradation in the proteasome.

Alzheimer's Disease (AD) is a neurodegenerative disorder characterized by the presence of β-Amyloid protein deposits in the core of neuritic plaques and abnormal neurofibrillary tangles in the brain of AD patients. The Amyloid β-protein (Aβ) is formed by two endoproteolytic cleavages of the Amyloid β protein precursor (AβPP), a large transmembrane type I protein. A protease termed β-secretase cleaves AβPP at the N-terminus of the Aβ domain to generate the soluble AβPP and the membrane anchored C-terminal fragments (CTFs). Then, a second secretase called γ-secretase, cuts CTFs within the transmembrane region to form Aβ, to form Aβ, which is secreted from the cells. The identification of compounds able to prevent or reduce this event has become an important goal for the research on the treatment of AD.

Also other diseases have been linked to the presence of beta Amyloid deposits in the brain. Some examples are MCI (mild cognitive impairment), Down's syndrome, Hereditary Cerebral Hemmorhage with Amyloidosis of the Dutch-Type, cerebral Amyloid angiopathy, other degenerative dementias, including dementias of mixed vascular and degenerative origin, dementia associated with Parkinson's disease, dementia associated with progressive supranuclear palsy, dementia associated with cortical basal degeneration, and diffuse Lewy body type Alzheimer's disease (see publication US20040132782).

BACE (β site AβPP cleaving enzyme) is an aspartyl protease with β-secretase activity. BACE is a type I integral membrane protein with a typical aspartyl protease motif in its luminal domain. BACE hydrolyzes AβPP specifically at the Met-Asp site, with an acidic pH optimum. BACE is highly expressed in the brain and it colocalizes with the intracellular sites of CTFs and Aβ production. BACE has become an important target for the development of therapeutic compounds against Alzheimer's Disease.

There are several factors that increase the expression and activity of BACE. Oxidant agents and oxidative products, such as H₂O₂ or HNE (4-hidroxynonenal), which is an aldehydic end product of polyinsaturated fatty acids, were shown to increase intracellular and secreted Aβ levels in neuronal and non neuronal cells (Paola *et al.* 2000; Misonou *et al.* 2001; Frederikse *et al.* 1996). Many studies have been carried out to determine the cellular mechanisms that underlie the Aβ overproduction. In 2002, Tamagno et al.(Oxidative Stress Increases Expression and Activity of BACE in NT2 Neurons, 2002, Neurobiol. Dis., 10, 279-288) demonstrated that oxidative stress induces BACE protein levels and activity, and this event is mediated by the oxidative product HNE. According to this study, exposure of NT₂ cells to oxidant agents did not influence AβPP expression. The effect of these agents on Aβ is related to an increase of BACE1 expression via transcriptional up regulation of BACE1 gene (Oxidative stress potentiates BACE1 gene expression and Aβ generation, Tong et al., 2004, J. Neural. Transm., 112(3):455-69).

The identification of compounds which are able to prevent the effect of oxidative agents has become an important goal of current research in Alzheimer's Disease. Among these compounds, dehydroepiandrosterone (DHEA) and its role in the CNS have been studied by Tamagno et al. (Dehydroepiandrosterone reduces expression and activity of BACE in NT2 neurons exposed to oxidative stress, Tamagno et al., 2003, Neurobiol. Dis., 14, 291-301). DHEA is an adrenal steroid that serves as a precursor to both androgens and estrogens and is synthesized from sterol precursors in the nervous system (Balieu 1981). DHEA is known to improve a variety of functional activities in the CNS, including increased memory and learning in different animal models (Vallée et al. 2001) and exerts protection against excitatory amino acids and Aβ neurotoxicity. In this study, it has been demonstrated that a pre-treatment with DHEA is able to decrease the expression, protein levels and activity of BACE induced in NT₂ neurons by oxidative agents, such as Asc/Fe and H₂O₂/Fe. This protection seems to be due to the antioxidant properties of the steroid, able to prevent the production of the end products of lipid oxidation, such as HNE. The oxidative stress products induce an increase of BACE protein levels and activity, and this induction is due to a gene overexpression, as has been demonstrated by quantitative PCR analysis. Decline of DHEA concentrations with ageing led to the suggestion that it could be implicated in longevity and that its progressive decrease can be related with some of the aging-related degenerative disorders, including AD. In conclusion, DHEA is able to prevent the oxidative stress-dependent Amyloidogenic processing of AβPP through the negative modulation of the expression and activity of BACE.

The expression of BACE has been localized in the brain, in particular in neurons, indicating that neurons are the major source of β-Amyloid peptides in the brain. Astrocytes, on the other hand, are known to be important for β-Amyloid clearance and degradation, for providing trophic support to neurons and for forming a protective barrier between β-Amyloid deposits and neurons. However, according to Rossner et al. (Alzheimer's disease β-secretase BACE1 is not a neuron specific enzyme, Rossner et al., J Neurobiochem. 2005, 92, 226-234), astrocytes may also represent an alternative cellular source of β-Amyloid peptides. The role of astrocytes in the pathogenesis of AD remains undetermined and may differ on a case to case instance due to dependence on a broad spectrum of interactive events in neurons, astrocytes and microglia.

### SUMMARY OF THE INVENTION

The present invention is related to a new family of phenyl-prenyl-ether derivatives of general formula (I). They have shown to exhibit an inhibitory effect on the enzymatic targets GSK-3, and most of them also on BACE, in *in vitro* assays. GSK-3, as detailed above, is known to play an important role in numerous diseases and conditions of very diverse nature, specially cognitive, neurodegenerative or neuronal diseases, and thus the inhibition of this enzyme is known to be a good therapeutic approach for the treatment of said diseases and conditions. Further, the inhibition of BACE enzyme, as detailed above, is also a good therapeutic target for the treatment of a number of diseases and conditions. Thus, taking into account that these enzymes are known to be involved in a variety of cognitive, neurodegenerative or neuronal diseases or disorders, and that their inhibition is known to help to prevent and treat these diseases, the compounds of formula (I) are useful for the prevention and/or treatment of cognitive, neurodegenerative or neuronal diseases or disorders.

Therefore, in a first aspect, the present invention is related to a novel compound of formula (I) (also referred to as the compound of the invention) 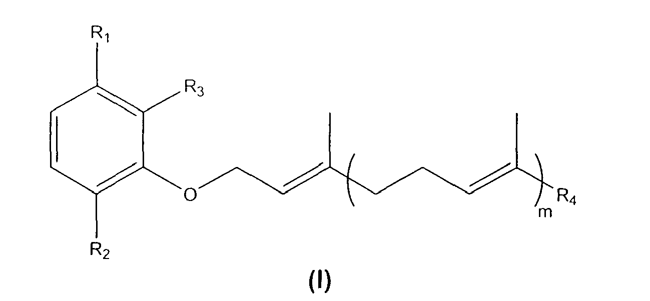 wherein
m is an integer selected from 0, 1, 2, 3, 4, 5 or 6;
R₁ is selected from hydrogen, -C(=O)OCH₂OCH₃, -(CH₂)ᵣC(=O)OR₅ and -(CH₂)ᵣC(=O)NHR₅, wherein
r is an integer selected from 0, 1, 2, 3 or 4; and
R₅ being selected from hydrogen, C₁-C₁₂ alkyl, C₂-C₁₂ alkenyl, C₂-C₁₂ alkynyl, and 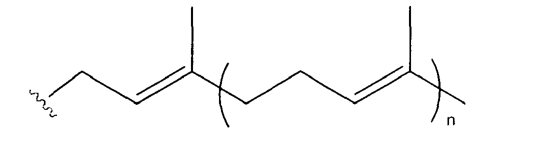 wherein n is an integer selected from 0, 1, 2, 3, 4, 5 or 6;
R₂ is selected from hydrogen, -NH₂, -NHC(=O)R₆ and -OR₆,
R₆ being selected from hydrogen, C₁-C₆ alkyl and 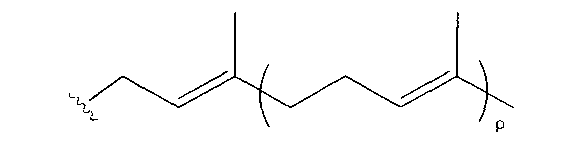 wherein p is an integer selected from 0, 1, 2, 3, 4, 5 or 6;
R₃ is selected from hydrogen and 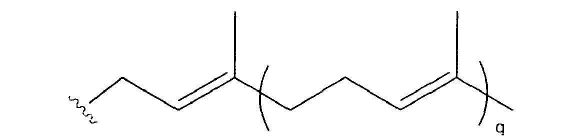 wherein q is an integer selected from 0, 1, 2, 3, 4, 5 or 6;
and R₄ is selected from -CH₃, -CH₂-CH₃, -CH₂-OH and -CH₂-CH₂-OH;
with the proviso that at least one of R₁ and R₂ is not hydrogen, and the provisos that:
when m is 0, R₂ and R₃ are hydrogen and R₄ is -CH₃, R₁ may not be -CH₂-C(=O)O-CH₂-CH₃;
when m is 0, R₁ and R₃ are hydrogen and R₄ is -CH₃, R₂ may not be -OH nor - OCH₃;
when m is 1, R₁ and R₃ are hydrogen and R₄ is -CH₃, R₂ may not be -OH nor-OCH₃;
when m is 2, R₁ and R₃ are hydrogen and R₄ is -CH₃, R₂ may not be -OH;
when m is 0, R₁ and R₃ are hydrogen, R₄ is -CH₃, R₂ may not be -O-CH₂-CH=C(CH₃)₂;
when m is 0, R₁ and R₃ are hydrogen, R₄ is -CH₃, R₂ may not be -NH₂;
and any pharmaceutically acceptable salts, solvates and prodrugs thereof.

The compounds of formula I may comprise asymmetric substituents, i.e. asymmetric substituents in R₁, R₂, R₃ and/or R₄, which may give raise the presence of different stereoisomers (enantiomer, stereoisomers, etc). The present invention comprises all such stereoisomers.

A further aspect of the present invention is a novel compound of formula (I) as defined above, for use as a medicament.

The present invention is further related to a pharmaceutical composition comprising at least one of the compounds of formula (I) as defined above, or salts, solvates or prodrugs thereof, and at least one pharmaceutically acceptable carrier, adjuvant and/or vehicle.

A further aspect of the present invention is a process for the preparation of a compound of formula (I) as defined above, comprising reacting the corresponding phenol of formula (A) 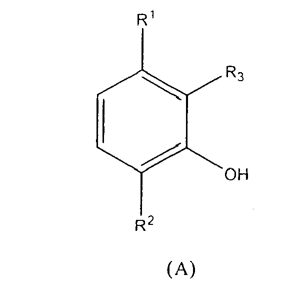 wherein R₁, R₂ and R₃ are as defined above; with a suitable unsaturated alkyl bromide of formula (B) 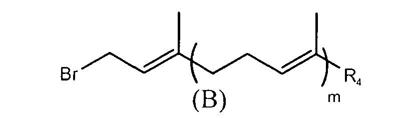 wherein m is as defined above;
in the presence of a base

Another aspect of the present invention is the use of a compound of formula (I) 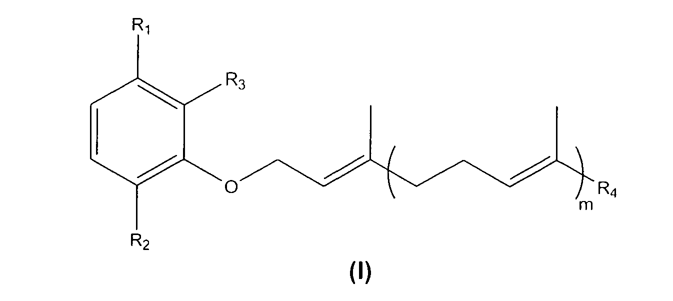 wherein
m is an integer selected from 0, 1, 2, 3, 4, 5 or 6;
R₁ is selected from hydrogen, -C(=O)OCH₂OCH₃, -(CH₂)ᵣC(=O)OR₅ and-(CH₂)ᵣC(=O)NHR₅, wherein
r is an integer selected from 0, 1, 2, 3 or 4; and
R₅ being selected from hydrogen, C₁-C₁₂ alkyl, C₂-C₁₂ alkenyl, C₂-C₁₂ alkynyl, and 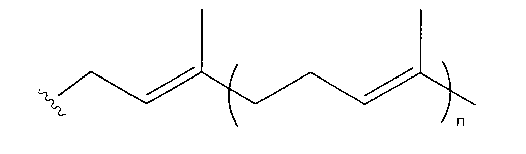 wherein n is an integer selected from 0, 1, 2, 3, 4, 5 or 6;
R₂ is selected from hydrogen, -NH₂, -NHC(=O)R₆ and -OR₆,
R₆ being selected from hydrogen, C₁-C₆ alkyl and 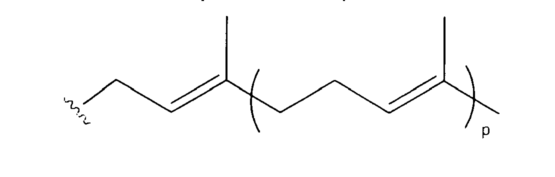 wherein p is an integer selected from 0, 1, 2, 3, 4, 5 or 6;
R₃ is selected from hydrogen and 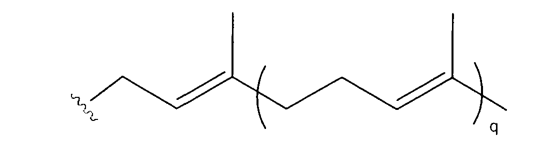 wherein q is an integer selected from 0, 1, 2, 3, 4, 5 or 6;
and R₄ is selected from -CH₃, -CH₂-CH₃, -CH₂-OH and -CH₂-CH₂-OH;
and any pharmaceutically acceptable salts, solvates and prodrugs thereof;
in the manufacture of a medicament for the treatment and/or profilaxis of a cognitive, neurodegenerative or neuronal disease or disorder.

In a further aspect, the present invention is related to a method of treating and/or preventing a cognitive, neurodegenerative or neuronal disease or disorder, which method comprises administering to a patient in need of such a treatment a therapeutically effective amount of at least one compound of formula (I) as defined in above or a pharmaceutical composition thereof.

### DETAILED DESCRIPTION OF THE INVENTION

In the above definition of compounds of formula (I) the following terms have the meaning indicated:

The term "C₁-C₁₂ alkyl" refers to a linear or branched hydrocarbon chain radical consisting of carbon and hydrogen atoms, containing no unsaturation, having one to twelve carbon atoms, and which is attached to the rest of the molecule by a single bond. Examples of alkyl groups include, but are not limited to alkyl groups such as methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, and hexyl, heptyl, and octyl. An alkyl group can be unsubstituted or substituted with one or two suitable substituents.

The term "C₂-C₁₂ alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl), hexenyl (e.g. 1-hexenyl, 2-hexenyl, 3-hexenyl, 4-hexenyl, 5-hexenyl), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl), 2-propyl-2-butenyl, 4,6-Dimethyl-oct-6-enyl. An alkenyl group can be unsubstituted or substituted with one or two suitable substituents.

The term "C₁-C₁₂ alkoxy" refers to a radical of the formula -ORa, wherein Ra is an alkyl radical as defined above, e. g., methoxy, ethoxy, propoxy, etc.

The term "alkoxymethyl ether" refers to a radical of formula -CH₂-O-R', wherein R' is an alkyl, alkenyl, aryl, aralkyl or trialkylsilyl radical as defined herein, such as methoxymethyl ether, 2-methoxyethoxymethyl ether, benzyloxymethyl ether, p-methoxybenzyloxymethyl ether, 2-(trimethylsilyl)ethoxymethyl ether.

The term "C₂-C₁₂ alkynyl" means a linear or branched hydrocarbon chain radical having one or more carbon-carbon triple bonds therein and from two to twelve carbon atoms, and which is attached to the rest of the molecule by a single bond. The triple bond of an alkynyl group can be unconjugated or conjugated to another unsaturated group. Suitable alkynyl groups include, but are not limited to alkynyl groups such as ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl), hexynyl (e.g. 1-hexynyl, 2-hexynyl, 3-hexynyl, 4-hexynyl, 5-hexynyl), methylpropynyl, 3-methyl-1-butynyl, 4-methyl-2-heptynyl , and 4-ethyl-2-octynyl. An alkynyl group can be unsubstituted or substituted with one or two suitable substituents.

The term "C₁-C₁₂ alkylamino" is intended to mean "C₁-C₁₂ monoalkylamino", and refers to an amino group attached to the rest of the molecule by a single bond, substituted with a single alkyl chain as defined above.

The term "C₁-C₁₂ dialkylamino" refers to an amino group attached to the rest of the molecule by a single bond, substituted with two alkyl chains, each one the same or different as defined above.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C₁₋₆ alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

According to a first aspect, the present invention is related to a novel compound of general formula (I) 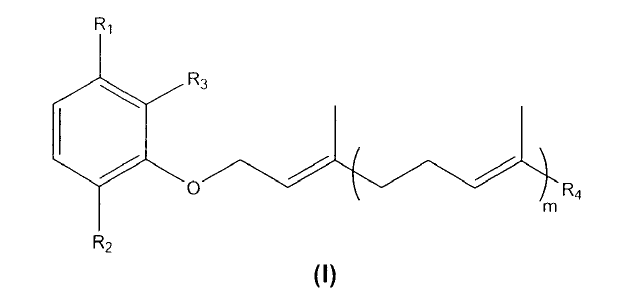 wherein
m is an integer selected from 0, 1, 2, 3, 4, 5 or 6;
R₁ is selected from hydrogen, -C(=O)OCH₂OCH₃, -(CH₂)ᵣC(=O)OR₅ and -(CH₂)ᵣC(=O)NHR₅, wherein
r is an integer selected from 0, 1, 2, 3 or 4; and
R₅ being selected from hydrogen, C₁-C₁₂ alkyl, C₂-C₁₂ alkenyl, C₂-C₁₂ alkynyl, and 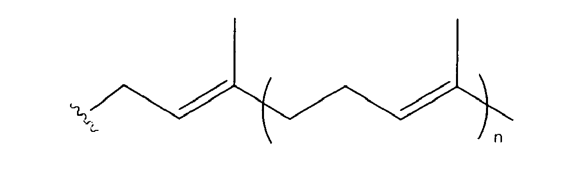 wherein n is an integer selected from 0, 1, 2, 3, 4, 5 or 6;
R₂ is selected from hydrogen, -NH₂, -NHC(=O)R₆ and -OR₆,
R₆ being selected from hydrogen, C₁-C₆ alkyl and 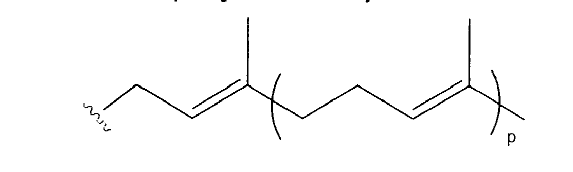 wherein p is an integer selected from 0, 1, 2, 3, 4, 5 or 6;
R₃ is selected from hydrogen and 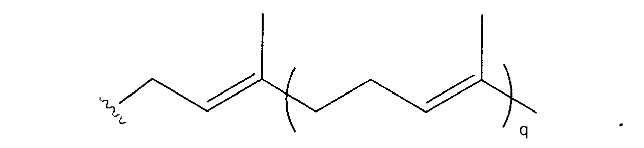 wherein q is an integer selected from 0, 1, 2, 3, 4, 5 or 6;
and R₄ is selected from -CH₃, -CH₂-CH₃, -CH₂-OH and -CH₂-CH₂-OH;
with the proviso that at least one of R₁ and R₂ is not hydrogen, and the provisos that:
when m is 0, R₂ and R₃ are hydrogen and R₄ is -CH₃, R₁ may not be -CH₂-C(=O)O-CH₂-CH₃;
when m is 0, R₁ and R₃ are hydrogen and R₄ is -CH₃, R₂ may not be -OH nor-OCH₃;
when m is 1, R₁ and R₃ are hydrogen and R₄ is -CH₃, R₂ may not be -OH nor-OCH₃;
when m is 2, R₁ and R₃ are hydrogen and R₄ is -CH₃, R₂ may not be -OH;
when m is 0, R₁ and R₃ are hydrogen, R₄ is -CH₃, R₂ may not be -O-CH₂-CH=C(CH₃)₂;
when m is 0, R₁ and R₃ are hydrogen, R₄ is -CH₃, R₂ may not be -NH₂;
and any pharmaceutically acceptable salts, solvates and prodrugs thereof.

A preferred group of compounds of formula (I) are those wherein R₁ is selected from hydrogen, -C(=O)OH, -C(=O)OCH₃, -C(=O)O-CH₂-CH₃, -C(=O)NHR₅ and C(=O)OR₅, wherein R₅ is 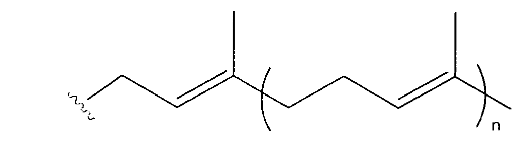 wherein n is an integer selected from 0, 1, 2 or 3.

Also preferred compounds are those wherein R₂ is selected from hydrogen,-OH, -OCH₃, -NH₂, -NHC(=O)CH₃, -OR₆, wherein R₆ is 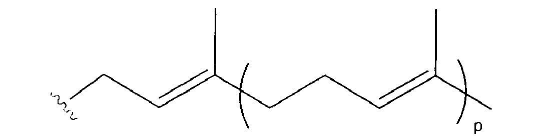 wherein p is an integer selected from 0, 1, 2 or 3.

A further group of preferred compounds are those wherein R₃ is hydrogen.

A further group of preferred compounds are those wherein R₄ is -CH₃.

Preferably, m is an integer selected from 0, 1, 2 or 3.

Preferred compounds of formula (I) are the following: 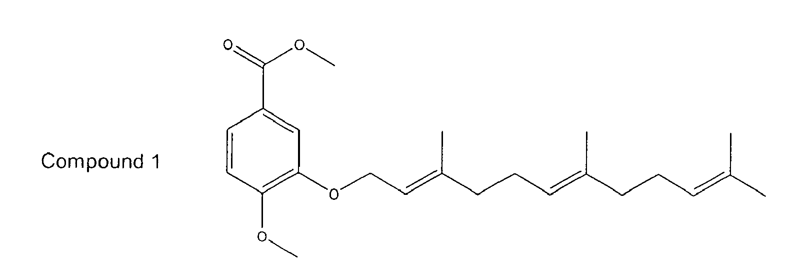 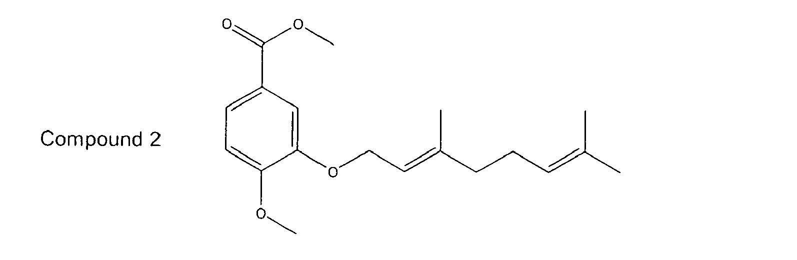 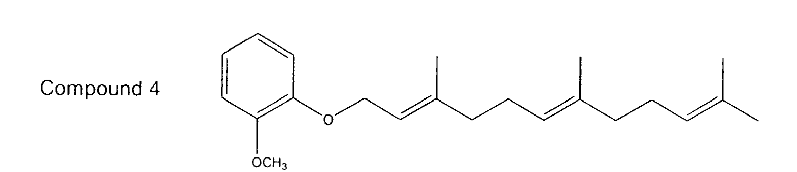 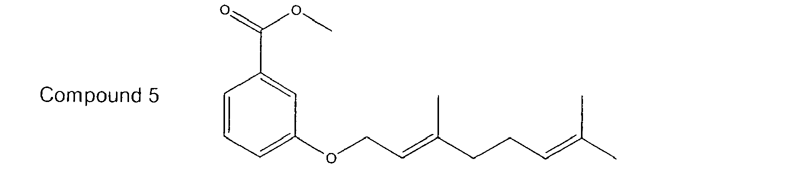 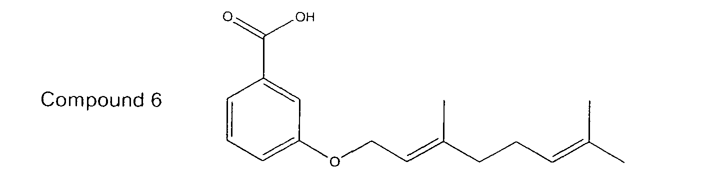 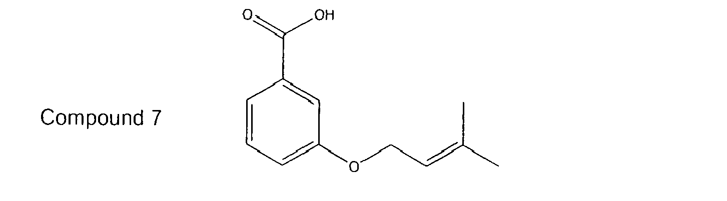 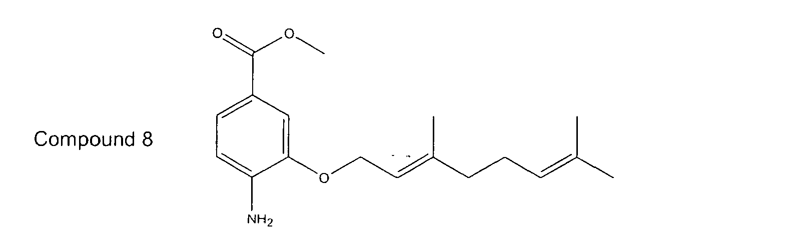 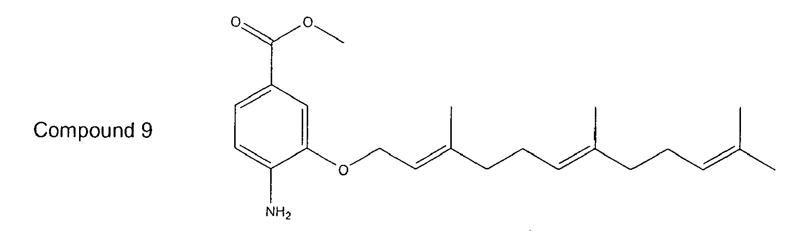 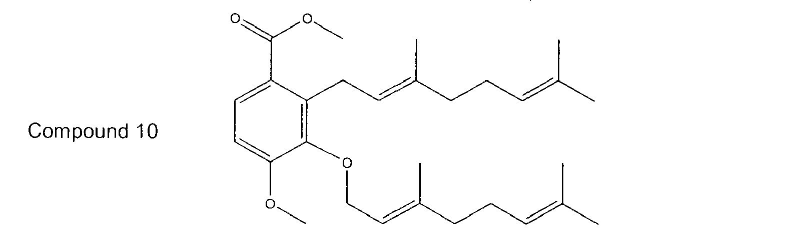 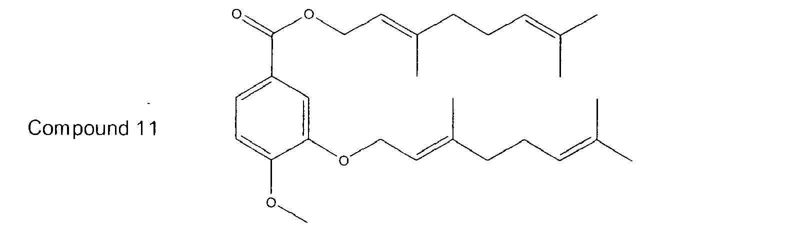 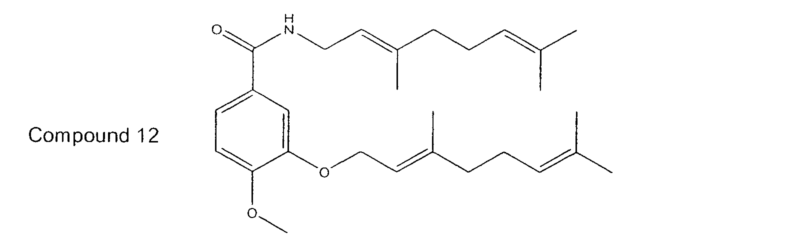 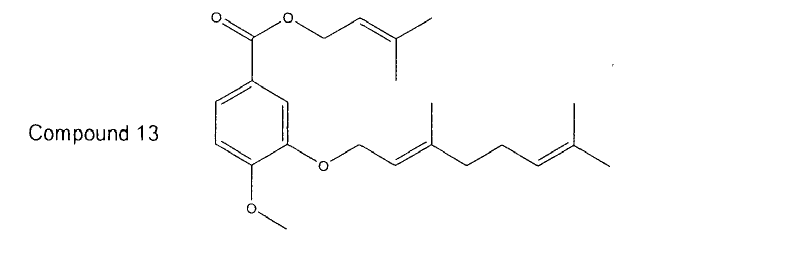 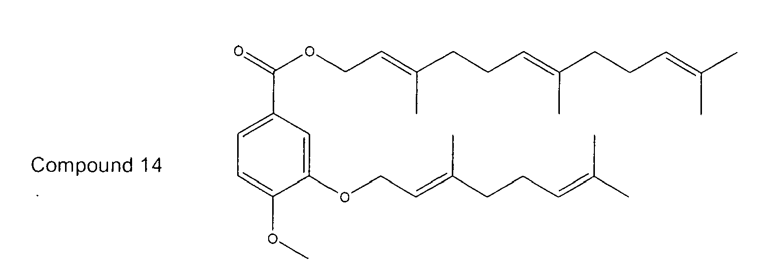 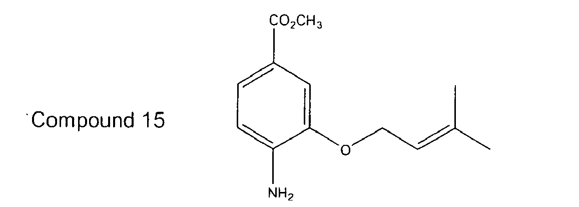 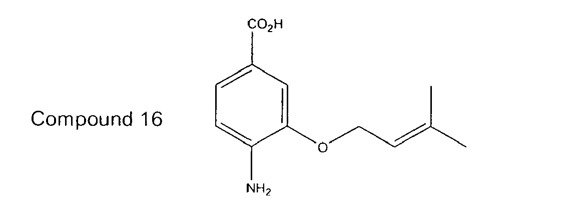 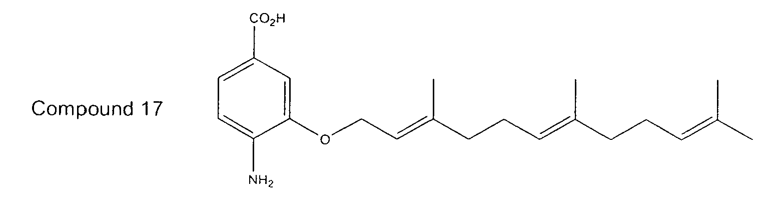 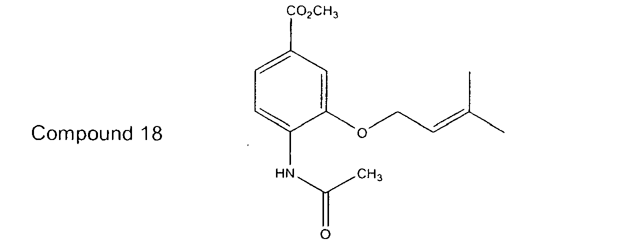 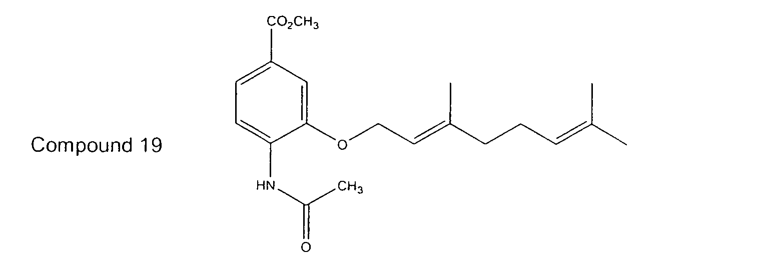 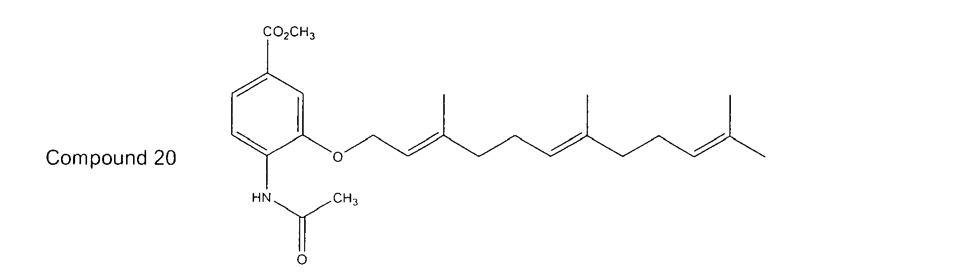 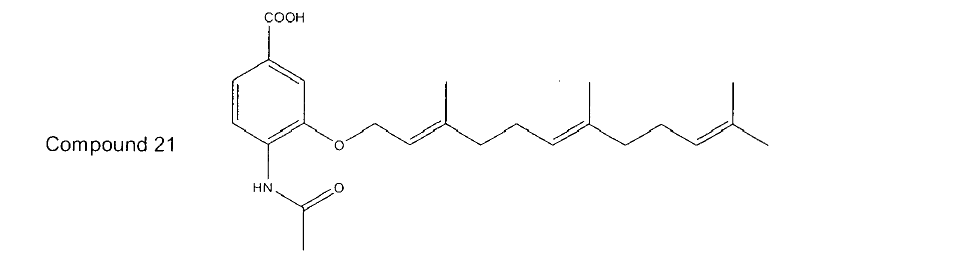 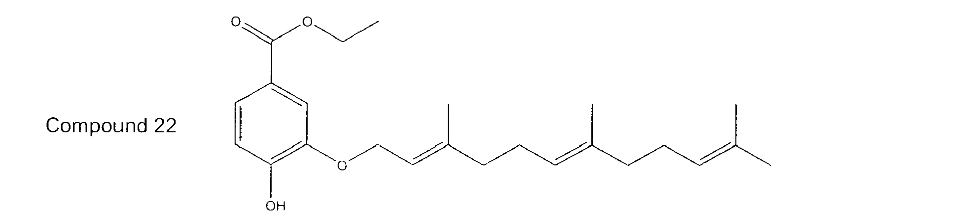 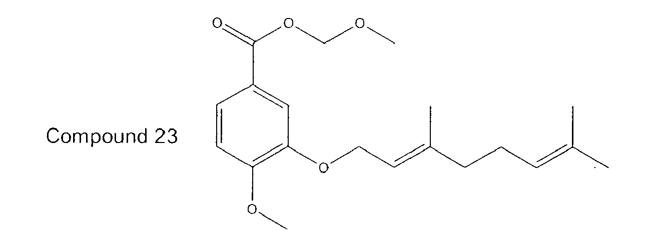 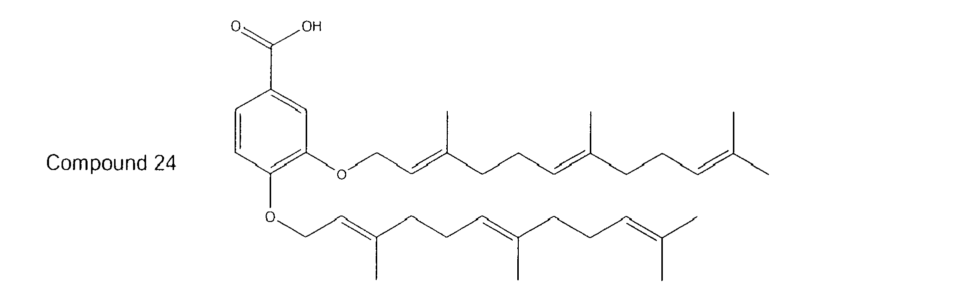 and pharmaceutically acceptable salts, solvates and prodrugs thereof.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates and prodrugs thereof" refers to salts, solvates, or prodrugs which, upon administration to the recipient are capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

The term "prodrug" as used in this application is defined here as meaning a chemical compound having undergone a chemical derivation such as substitution or addition of a further chemical group to change (for pharmaceutical use) any of its physico-chemical properties, such as solubility or bioavailability, e.g. ester and ether derivatives of an active compound that yield the active compound per se after administration to a subject. Examples of well known methods of producing a prodrug of a given acting compound are known to those skilled in the art and can be found e.g. in Krogsgaard-Larsen et al., Textbook of Drugdesign and Discovery, Taylor & Francis (April 2002).The term "solvate" according to this invention is to be understood as meaning any form of the compound of the invention which has another molecule (most likely a polar solvent) attached to it via non-covalent bonding. Examples of solvates include hydrates and alcoholates, e.g. methanolate.

Particularly favoured prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The preparation of salts, solvates and produrgs can be carried out by methods known in the art. It will be appreciated that non-pharmaceutically acceptable salts, solvates or prodrugs also fall within the scope of the invention since those may be useful in the preparation of pharmaceutically acceptable salts, solvates or prodrugs.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of formula (I) according to the present invention or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

Another aspect of the present invention is a compound of formula (I) as defined above, for use as a medicament.

The present invention further provides pharmaceutical compositions comprising at least a novel compound of formula (I) of the present invention, or pharmaceutically acceptable salts, solvates or prodrugs thereof and at least one pharmaceutically acceptable carrier, adjuvant, and/or vehicle, for administration to a patient.

The term "carrier, adjuvant and/or vehicle" refers to a molecular entities or substances with which the active ingredient is administered. Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form. Suitable dosage forms for oral administration may be tablets or capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the novel compounds of formula (I) or compositions of the present invention may be by any suitable method, such as intravenous infusion, oral preparations, and intraperitoneal and intravenous administration. Oral administration is preferred because of the convenience for the patient and the chronic character of many of the diseases to be treated.

The novel compounds and compositions of this invention may be used with other drugs to provide a combination therapy. The other drugs may form part of the same composition, or be provided as a separate composition for administration at the same time or at different time.

An additional aspect is the use of a compound of formula (I) 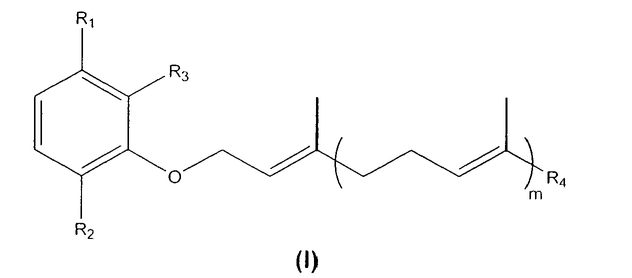 wherein
m is an integer selected from 0, 1, 2, 3, 4, 5 or 6;
R₁ is selected from hydrogen, -C(=O)OCH₂OCH₃, -(CH₂)ᵣC(=O)OR₅ and -(CH₂)ᵣC(=O)NHR₅, wherein
r is an integer selected from 0, 1, 2, 3 or 4; and
R₅ being selected from hydrogen, C₁-C₁₂ alkyl, C₂-C₁₂ alkenyl, C₂-C₁₂ alkynyl, and 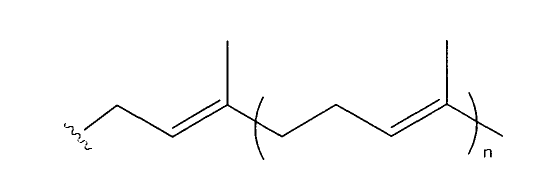 wherein n is an integer selected from 0, 1, 2, 3, 4, 5 or 6;
R₂ is selected from hydrogen, -NH₂, -NHC(=O)R₆ and -OR₆,
R₆ being selected from hydrogen, C₁-C₆ alkyl and 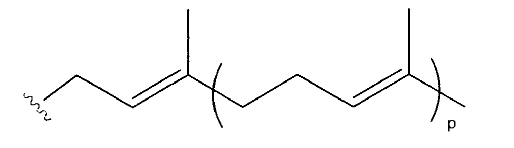 wherein p is an integer selected from 0, 1, 2, 3, 4, 5 or 6;
R₃ is selected from hydrogen and 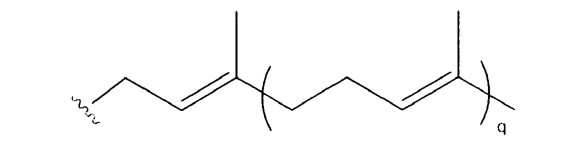 wherein q is an integer selected from 0, 1, 2, 3, 4, 5 or 6;
and R₄ is selected from -CH₃, -CH₂-CH₃, -CH₂-OH and -CH₂-CH₂-OH;
and any pharmaceutically acceptable salts, solvates and prodrugs thereof;
in the manufacture of a medicament for the treatment and/or profilaxis of a cognitive, neurodegenerative or neuronal disease or disorder.

Within the frame of the present invention, "a cognitive, neurodegenerative or neuronal disease or disorder" refers to any disease, disorder or condition selected from, but not limited to, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding, such as cerebral bleeding due to solitary cerebral amyloid angiopathy, mild cognitive impairment, Hereditary Cerebral Hemmorhage with Amyloidosis of the Dutch-Type, cerebral Amyloid angiophathy, ischaemia, brain injury, especially traumatic brain injury, Down's syndrome, Lewy body disease, inflammation and chronic inflammatory diseases

Preferred diseases or disorders are diabetes, chronic neurodegenerative conditions including dementias such as Alzheimer's disease and Parkinson's disease, Huntington's Disease, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding, mild cognitive impairment, ischaemia, brain injury, especially traumatic brain injury, inflammation and chronic inflammatory diseases.

Especially preferred diseases are Alzheimer's Disease, Parkinson's Disease, multiple sclerosis, stroke, epilepsy, mood disorders, ischaemia, brain injury and chronic inflammatory diseases.

The compounds of formula (I) according to the present invention may be synthetically prepared starting from commercially available compounds; the compounds may be synthesized by direct alkylation of differently substituted commercially available phenols with the corresponding unsaturated alkyl bromides, and eventual subsequent derivatisation. 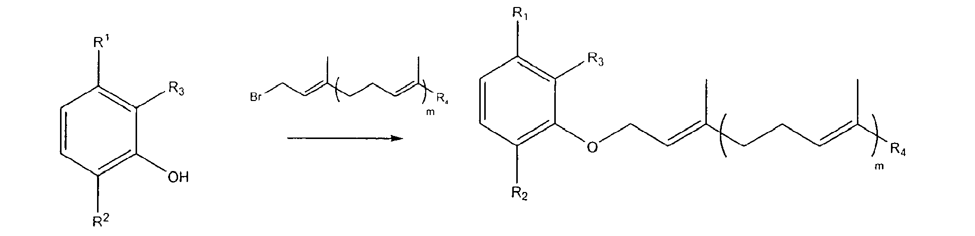 wherein R₁, R₂, R₃, R₄ and m are as defined above.

Therefore, according to a further aspect, the present invention refers to a process for the preparation of a compound of formula (I) as defined above, comprising reacting the corresponding phenol of formula (A) 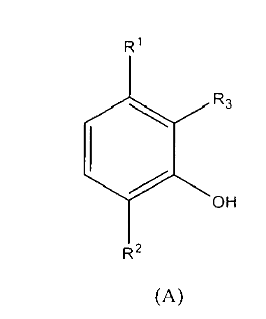 wherein R₁, R₂ and R₃ are as defined above; with a suitable unsaturated alkyl bromide of formula (B) 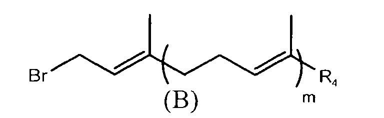 wherein m is as defined above; in the presence of a base.

Another aspect of the present invention is a method of treating and/or preventing a cognitive, neurodegenerative or neuronal disease or disorder, which method comprises administering to a patient in need of such a treatment a therapeutically effective amount of at least one compound of formula (I) as defined above or a pharmaceutical composition thereof.

The term "cognitive, neurodegenerative or neuronal disease or disorder" shall be interpreted as indicated above.

The disease or disorder is preferably selected from, but not limited to, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding, such as cerebral bleeding, due to solitary cerebral amyloid angiopathy, mild cognitive impairment, Hereditary Cerebral Hemmorhage with Amyloidosis of the Dutch-Type, cerebral Amyloid angiophathy, ischaemia, brain injury, especially traumatic brain injury, Down's syndrome, Lewy body disease, inflammation and chronic inflammatory diseases.

Generally a "therapeutically effective amount" of the compound of the invention or a pharmaceutical composition thereof will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

In the following, the present invention is further illustrated by examples. They should in no case be interpreted as a limitation of the scope of the invention as defined in the claims.

### EXAMPLES

### Preparation

Following the above-indicated general reaction scheme, the following compounds were obtained:

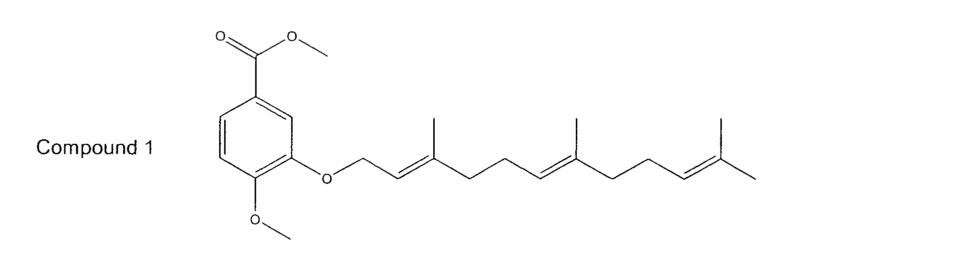

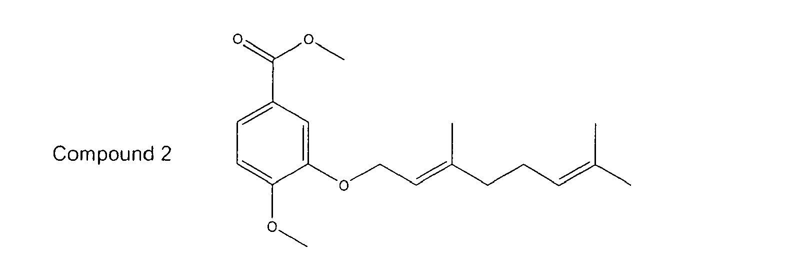

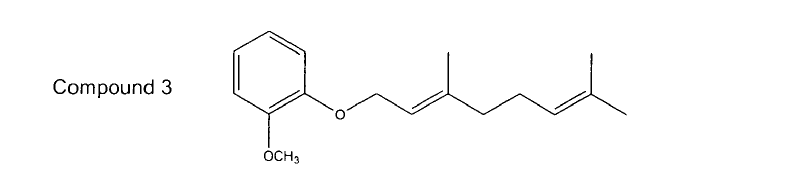

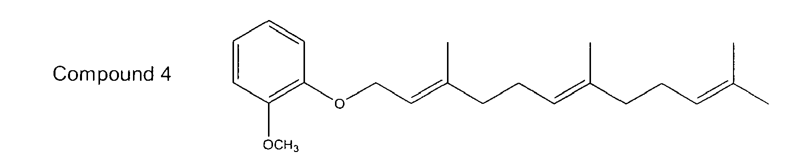

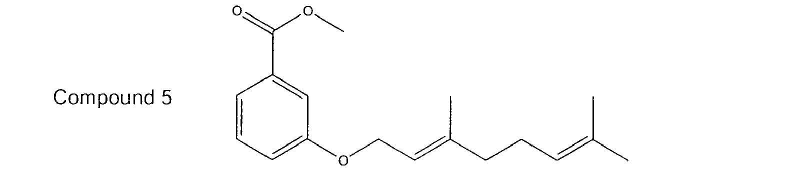

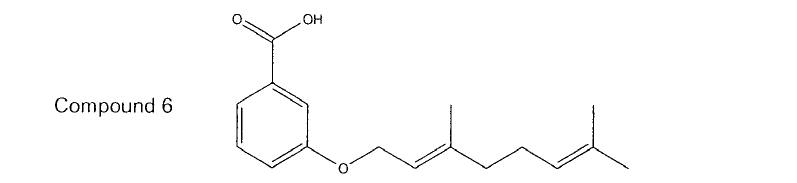

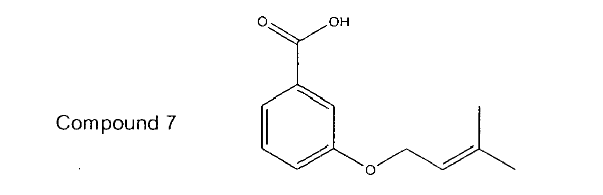

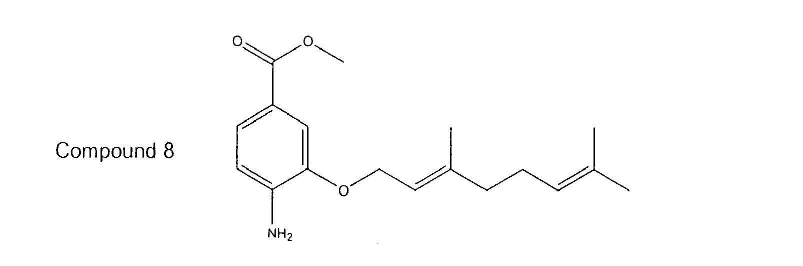

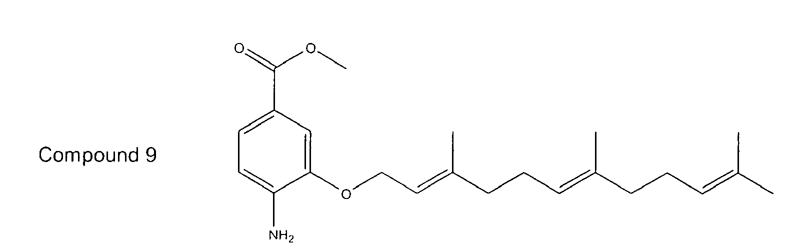

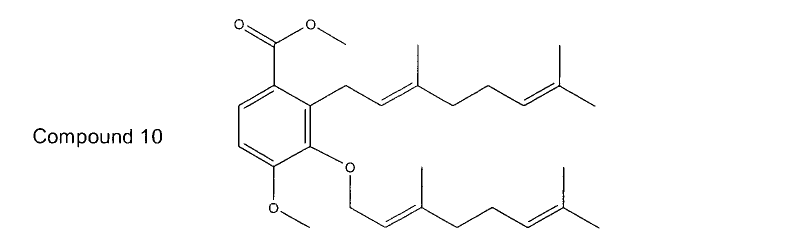

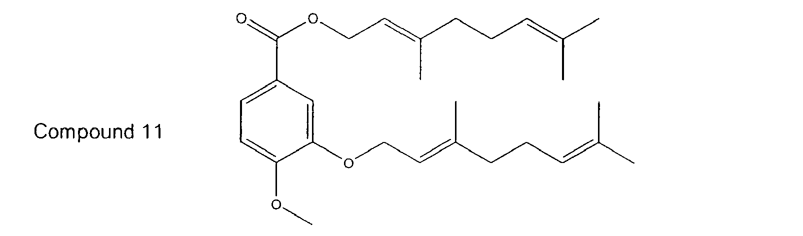

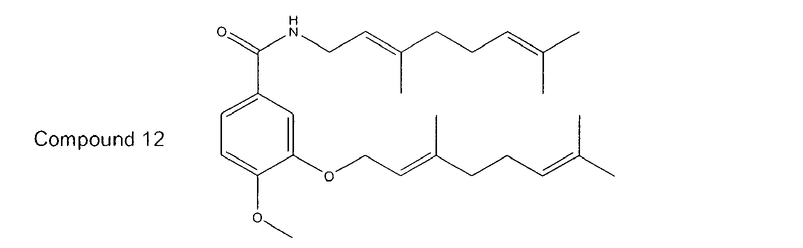

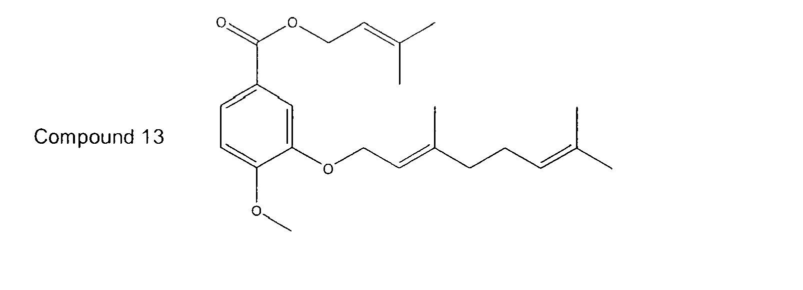

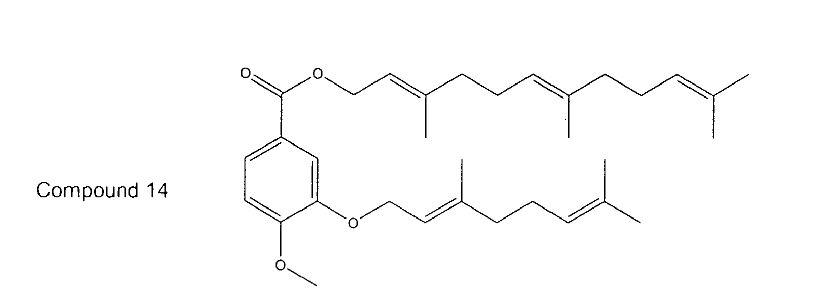

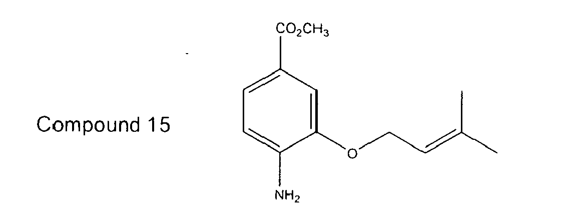

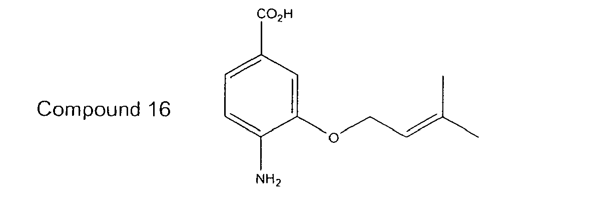

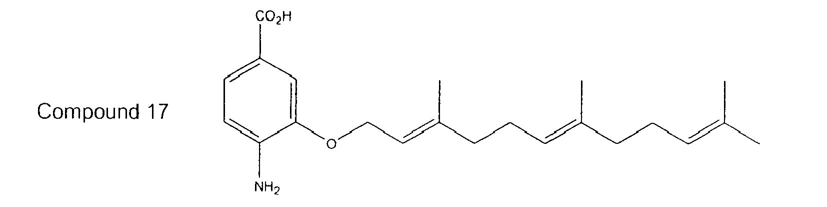

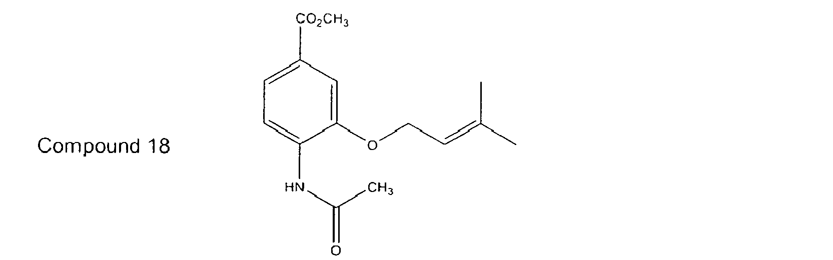

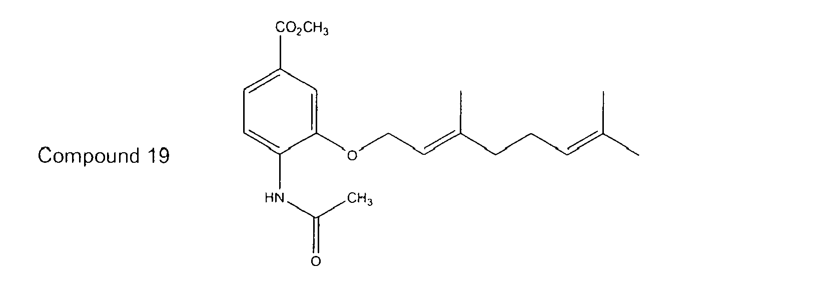

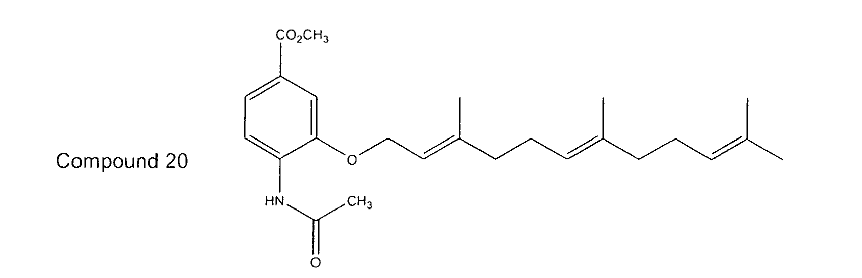

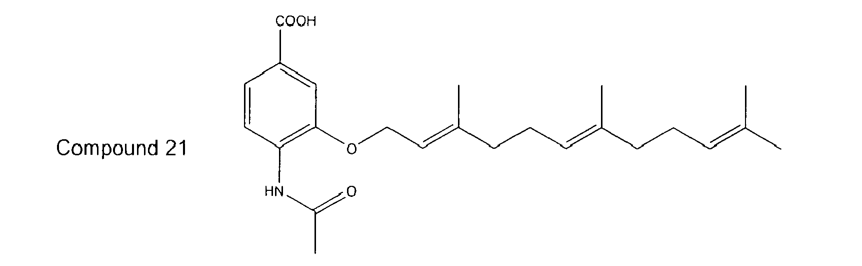

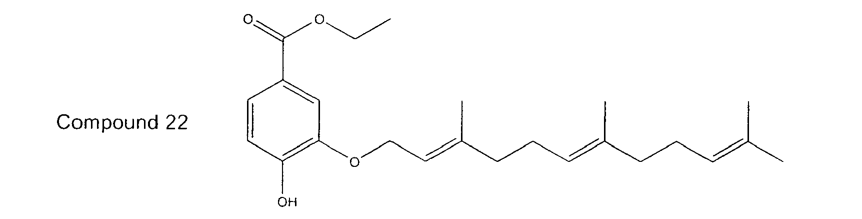

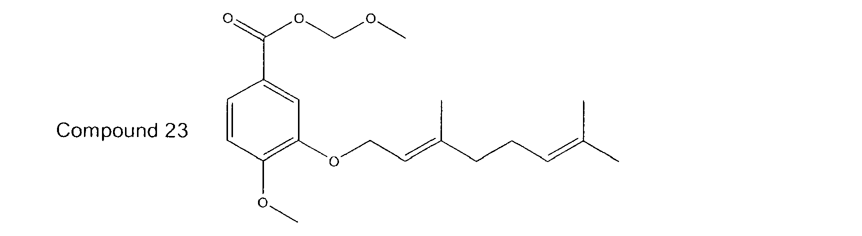

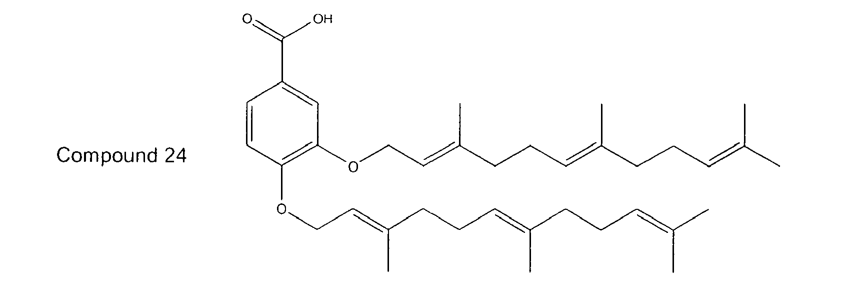

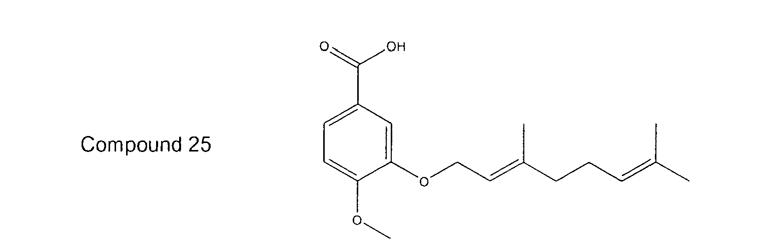

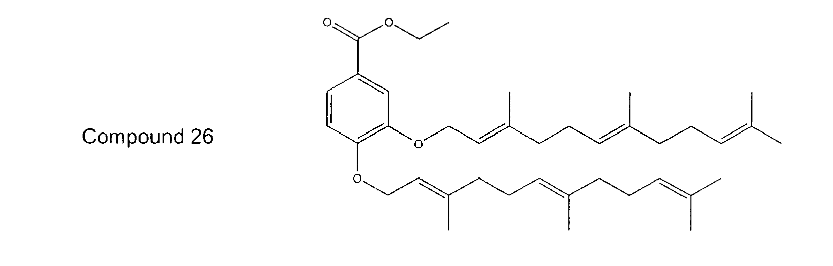

The detailed preparation of some of the compounds is described hereinafter:

### Example 1: Preparation of Compound 8

### 4-Amino-3-(3,7-dimethyl-octa-2,6-dienyloxy)-benzoic acid methyl ester

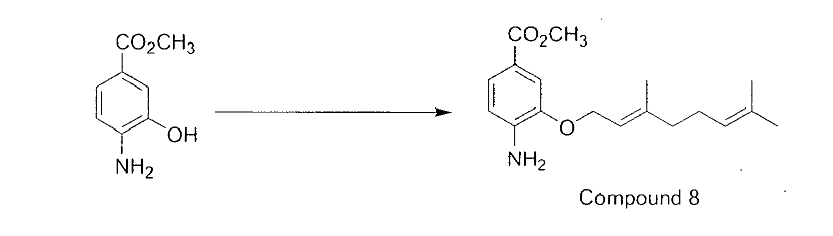

A solution of 4-amino-3-hydroxy-benzoic acid methyl ester (500 mg, 3.00 mmol) in anhydrous tetrahydrofuran (3 mL) was added under nitrogen to a suspension of sodium hydride (60% in mineral oil, 132 mg, 3.30 mmol) in dry tetrahydrofuran (5 mL). The mixture was stirred at room temperature for 20 minutes followed by the addition of a suspension of tetrabutylammonium iodide (240 mg, 0.65 mmol) and 18-crown-6 (4 mg, 0.02 mmol) in tetrahydrofuran anhydrous (4 mL) and a solution of geranyl bromide (716 mg, 3.30 mmol) in dry tetrahydrofuran (2 mL). The reaction mixture was stirred at room temperature for 18 hours. Solvent was removed in vacuo and the residue was distributed into water and methylene chloride. The organic layer was washed with a saturated solution of sodium chloride, dried with anhydrous sodium sulfate and evaporated. Flash chromatography (SiO₂, ethyl acetate/ hexanes 1:10) yielded 525 mg (58% yield) of pure material as a white solid.
**¹H NMR** (400 MHz, CDCl₃):
7.52, 7.46, 6.64, 5.49, 5.09, 4.60, 4.26, 3.85, 2.10, 1.74, 1.67, 1.60
**¹³C NMR** (100MHz, CDCl₃):
167.56, 145.54, 141.70, 141.56, 132.00, 124.22, 123.99, 119.57, 119.53, 113.32, 112.74, 65.59, 51.85, 39.77, 26.54, 25.88, 17.92, 16.88

### Example 2: Preparation of Compound 19

### 4-Acetylamino-3-(3,7-dimethyl-octa-2,6-dienyloxy)-benzoic acid methyl ester -

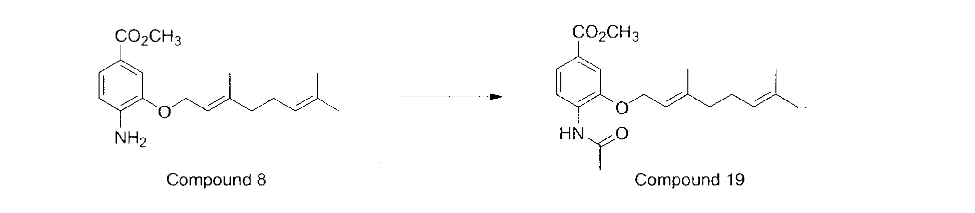

To a solution of **Compound 8** [see Example 1] (120 mg, 0.40 mmol) in anhydrous methylene chloride (2 mL) was added pyridine (39 µl, 0.48 mmol) and acetic anhydride (49 µL, 0.51 mmol). The reaction mixture was stirred at room temperature for 18 hours, diluted with methylene chloride and washed sequentally with 1N HCl and a saturated solution of sodium chloride. The organic layer was dried with anhydrous sodium sulfate and evaporated affording the desired material as a white solid (130 mg, 95% yield).
**¹H NMR** (400 MHz, CDCl₃):
8.42, 7.95, 7.61, 7.51, 5.43, 5.04, 4.62, 3.85, 2.18, 2.07, 1.74, 1.63, 1.57
**¹³C NMR** (100MHz, CDCl₃):
168.26, 166.60, 146.16, 142.15, 132.12, 131.74, 124.62, 123.44, 123.07, 118.49, 118.39, 111.86, 65.70, 51.86, 39.41, 26.10, 25.52, 24.89, 17.57, 16.56

### Example 3: Preparation of Compound 9

### 4-Amino-3-(3,7,11-trimethyl-dodeca-2,6,10-trienyloxy)-benzoic acid methyl ester

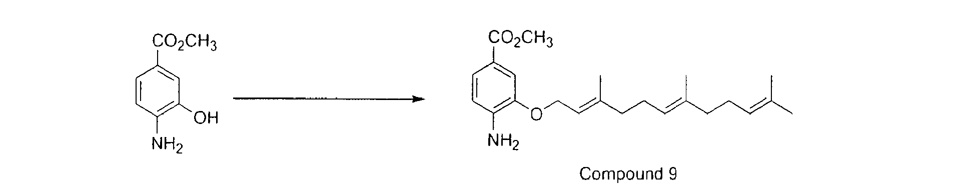

The compound was synthesized from 4-amino-3-hydroxy-benzoic acid methyl ester (500 mg, 3.0 mmol) and farnesyl bromide (941 mg, 3.3 mmol) following the procedure described for the preparation of 4-amino-3-(3,7-dimethyl-octa-2,6-dienyloxy)-benzoic acid methyl ester (**Compound 8**). Pure **Compound 9** (650 mg, 58% yield) was isolated as a colorless oil after purification by flash chromatography (SiO₂, ethyl acetate/hexanes 1:16).
**¹H NMR** (400 MHz, CDCl₃):
7.53, 7.56, 6.64, 5.49, 5.09, 4.60, 4.22, 3.85, 2.15-1.95, 1.75, 1.68, 1.60
**¹³C NMR** (100MHz, CDCl₃):
167.56, 145.54, 141.19, 141.60, 135.64, 131.51, 124.54, 124.22, 123.88, 119.56, 113.31, 112.72, 65.59, 51.85, 39.91, 39.79, 26.95, 26.47, 25.91, 17.91, 16.91, 16.24

### Example 4: Preparation of Compound 17

### 4-Amino-3-(3,7,11-trimethyl-dodeca-2,6,10-trienyloxy)-benzoic acid

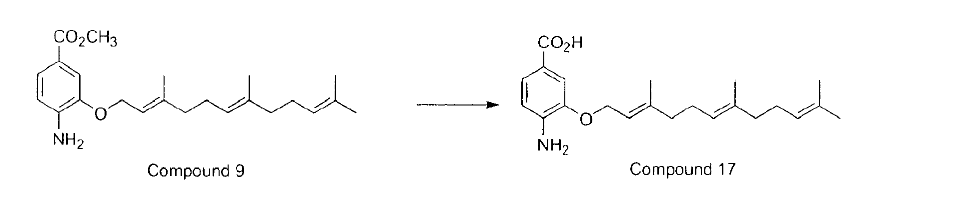

To a solution of 4-amino-3-(3,7,11-trimethyl-dodeca-2,6,10-trienyloxy)-benzoic acid methyl ester [**Compound 9**] (165 mg, 0.4 mmol) in a mixture of tetrahydrofuran (2 mL) and methanol (2 mL) a solution of lithium hydroxide monohydrate (280 mg, 6.7 mmol) in water (2 mL) was added. The reaction mixture was stirred at room temperature for 18 hours, diluted with water and adjusted to pH 4 with 1 N HCl and extracted twice with methylene chloride. The combined organic layers were washed with a saturated solution of sodium chloride, dried with anhydrous sodium sulfate and evaporated affording the pure acid **Compound 17** as a white wax-like solid (160 mg) with a quantitative yield.
**¹H NMR** (400 MHz, CDCl₃):
7.64, 7.53, 6.68, 5.51, 5.10, 4.63, 2.12, 1.99, 1.77, 1.69, 1.62, 1.61
**¹³C NMR** (100MHz, CDCl₃):
172.46, 145.20, 142.23, 141.48, 135.40, 131.26, 125.00, 124.28, 123.62, 119.21, 118.33, 113.01, 112.85, 65.35, 39.64, 39.53, 26.68, 26.21, 25.65, 17.64, 16.66, 15.99

### Example 5: Preparation of Compound 21

### 4-Acetylamino-3-(3,7,11-trimethyl-dodeca-2,6,10-trienyloxy)-benzoic acid

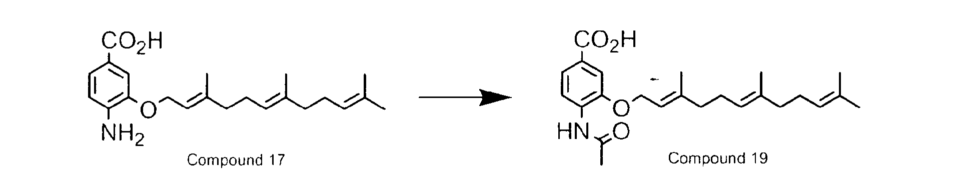

The reaction of N-acetylation of 4-amino-3-(3,7,11-trimethyl-dodeca-2,6,10-trienyloxy)-benzoic acid [**Compound 17**] (105 mg, 0.29 mmol) was performed employing the same synthetic procedure previously described for the preparation of 4-acetylamino-3-(3,7-dimethyl-octa-2,6-dienyloxy)-benzoic acid methyl ester [**Compound 19**]. Wax-like solid (94 mg, 80% yield)
**¹H NMR** (400 MHz, CDCl₃):
10.29, 8.49, 7.99, 7.75, 7.60, 5.48, 5.08, 4.67, 2.24, 2.14, 2.04, 1.96, 1.79, 1.67, 1.60, 1.59
**¹³C NMR** (100MHz, CDCl₃):
171.42, 168.58, 146.24, 142.49, 135.56, 132.88, 131.31, 124.19, 124.13, 123.98, 123.43, 118.54, 118.46, 112.31, 65.87, 39.64, 39.55, 26.66, 26.16, 25.63, 25.01, 17.63, 16.72, 15.99

### Example 6: Preparation of Compound 2

### 3-(3,7-Dimethyl-octa-2,6-dienyloxy)-4-methoxy-benzoic acid methyl ester

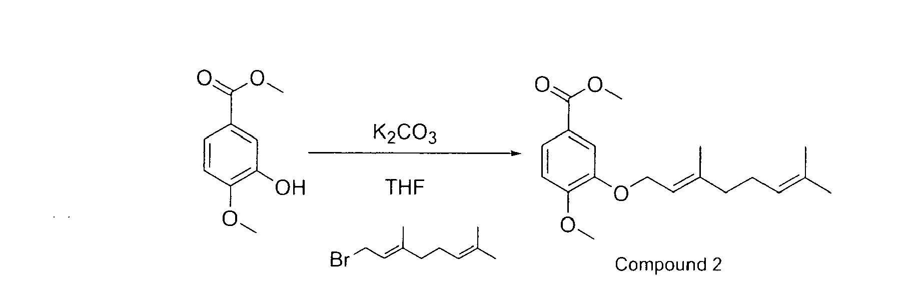

To a solution of 3-Hydroxy-4-methoxy-benzoic acid methyl ester (2 g, 11 mmol) in 20 mL of dimethylformamide (DMF), K₂CO₃ (3.8 g, 27.5 mmol) was added and the resulting mixture was stirred for 45 minutes. Geranyl bromide (3.6 g, 16.5 mmol,) was then added and the mixture was refluxed for 4 hours. After evaporation of the solvent under reduced pressure, water was added (250 mL) and the mixture was extracted with dichloromethane (DCM) (2 x 100 mL). The combined organic extracts were washed with saturated NaCl solution, dried (Na₂SO₄) and the solvent evaporated under reduced pressure to give the title compound (1.5 g, 43 % yield).
**¹H-NMR** (CDCl₃, 400MHz, δ ppm): 7.65 (dd, 1H, *J* = 8.4 Hz, *J* = 2.2 Hz), 7.55 (d, 1H, *J* = 8.4 Hz), 6.86 (d, 1H, *J* = 8.4 Hz), 5.52-5.50 (m, 1H), 5.09-5.05 (m, 1H), 4.65 (m, 2H), 3.91 (s, 3H), 3.90 (s, 3H), 2.11-2.04 (m, 4H), 1.75 (s, 3H), 1.67 (s, 3H), 1.65 (s, 3H).
**¹³C-NMR** (CDCl₃, 100MHz, δ ppm): 167.1, 153.0, 148.0, 141.5, 132.0, 124.0, 123.7, 122.7, 119.5, 114.0, 110.6, 66.1, 56.2, 52.1, 39.8, 26.5, 25.8, 17.8, 17.0.

### Example 7: Preparation of Compound 25

### 3-(3,7-Dimethyl-octa-2,6-dienyloxy)-4-methoxy-benzoic acid

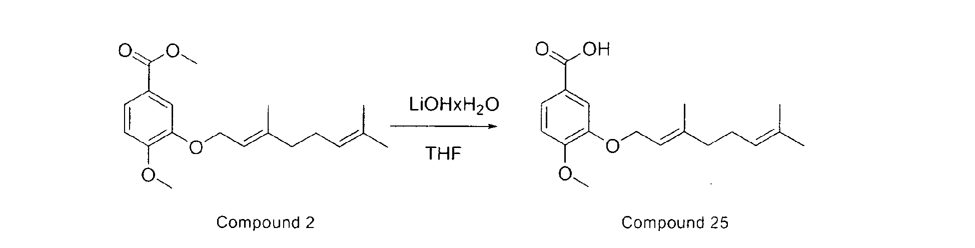

To a solution of 3-(3,7-dimethyl-octa-2,6-dienyloxy)-4-methoxy-benzoic acid [**Compound 25**, see example 6] (1.2 g, 3.7 mmol) in MeOH (5.5 mL), LiOH x H₂O (1.2 g, 28.3 mmol) was added and the resulting white suspension was stirred for 3 days. After evaporation of the solvent the reaction mixture was acidified with 0.1 M HCl to pH = 3-4 and the resulting white suspension was extracted with DCM (3 x 25 mL). The combined extracts were washed with saturated NaCl solution (50 mL), dried (Na₂SO₄) and the solvent evaporated under reduced pressure to give thetitle compound (1.1 g, 95%) as a white solid.
**¹H-NMR** (CDCl₃, 400MHz, δ ppm): 7.73 (m, 1 H), 7.60 (bs, 1 H), 6.86 (m, 1 H), 5.51-5.49 (m, 1H), 5.09-5.05 (m, 1H), 4.63 (m, 2H), 3.91 (s, 3H), 2.09-2.05 (m, 4H), 1.73 (s, 3H), 1.65 (s, 3H), 1.58 (s, 3H).
**¹³C-NMR** (CDCl₃, 100MHz, δ ppm): 172.0, 153.7, 148.0, 141.4, 131.7, 124.3, 123.8, 123.2, 119.1, 114.1, 110.3, 65.8, 59.4, 55.9, 39.6, 26.3, 25.6, 17.6, 16.0.

### Example 8: Preparation of Compound 11

### 3-(3,7-Dimethyl-octa-2,6-dienyioxy)-4-methoxy-benzoicacid37-dimethyl-octa-26-dienyl ester

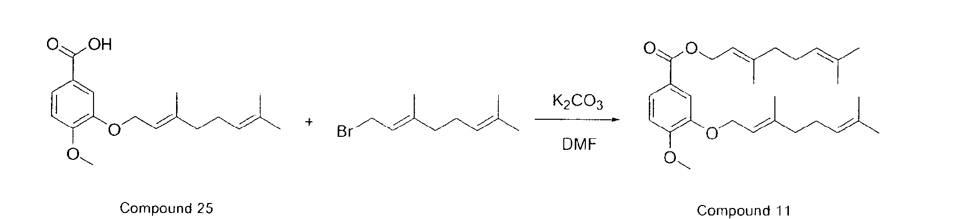

To a solution of 3-(3,7-dimethyl-octa-2,6-dienyloxy)-4-methoxy-benzoic acid [**Compound 25**, see example 6] (300 mg, 0.99 mmol) in DMF (5 mL), was added K₂CO₃ (169 mg, 0.99 mmol) and the resulting mixture was stirred for 10 minutes. Geranyl bromide (0.15 mL, 0.99 mmol) was then added and the mixture was stirred for seven hours at room temperature. After evaporation of the solvent under reduced pressure water was added (100 mL) and the mixture was extracted with diethyl ether (3 x 50 mL). The combined organic extracts were dried (Na₂SO₄) and the solvent evaporated under reduced pressure to give the title compound (224 mg, 51%).
**¹H-NMR** (CDCl₃, 400MHz, δ): 7.67 (dd, 1H, *J* = 8.4 Hz y *J* = 2 Hz), 7.55 (d, 1H, *J* = 2 Hz), 6.87 (1H, *J* = 8.4 Hz), 5.52-5.44 (m, 2H), 5.11-5.06 (m, 2H), 4.81 (d, 2H), 4.66 (d, 2H), 3.91 (s, 3H), 2.12-2.04 (m, 8H), 1.76 (6H), 1.67 (s, 3H), 1.65 (s, 3H), 1.60 (s, 3H), 1.58 (s, 3H)
**¹³C-NMR** (CDCl₃, 100MHz, δ ppm): 166.7, 153.5, 147.9, 142.22, 141.4, 132.0, 131.9, 124.0, 123.9, 123.6, 123.0, 119.5, 118.8, 113.9, 110.5, 66.0, 62.0, 56.2, 40.0, 39.7, 26.5, 26.0, 25.8, 18.0, 17.0

### Example 9: Preparation of Compound 12

### N-(3,7-Dimethyl-octa-2,6-dienyl)-3-(3,7-dimethyl-octa-2,6-dienyloxy)-4-methoxybenzamide

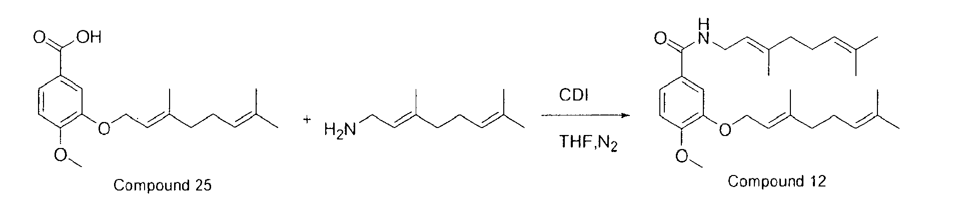

To a solution of 3-(3,7-dimethyl-octa-2,6-dienyloxy)-4-methoxy-benzoic acid [**Compound 25**, see Example 6] (300 mg, 0.99 mmol) in dry tetrahydrofurane (THF) (5 mL) was added 1,1 1'-carbonydiimidazol (CDI) (168 mg, 1.04 mmol) and the resulting mixture was stirred for 12 h at room temperature. A solution of geranylamine (92 mg, 0.60 mmol) in THF (2 mL) was added and the resulting amber solution stirred for further 7 hours. After evaporation of the solvent under reduced pressure water (100 mL) was added and the mixture was extracted with dichloromethane (3 x 50 mL). The combined organic extracts were dried (Na₂SO₄) and the solvent removed under reduced pressure to give the title compound (290 mg, 67%) as a brown solid.
**¹H-NMR** (CDCl₃, 400MHz, δ): 7.42 (d, 1H, *J* = 2 Hz), 7.25 (dd, 1H, *J* = 8, *J* = 2 Hz), (6.85, d, 1H, *J* = 8 Hz), 5.90-5.88 (m, 1H), 5.54-5.51 (m, 1H), 5.31-5.28 (m,1H), 5.09-5.07 (m, 2H), 4.66 (d, 2H), 4.05-4.02 (m, 2H), 3.92 (s, 3H), 2.12-2.06 (m, 8H), 1.74 (s, 3H), 1.72 (s, 3H), 1.68 (s, 3H), 1.66 (s, 3H), 1.60 (s, 3H), 1.59 (s, 3H).
**¹³C-NMR** (CDCl₃, 100MHz, δ ppm): 167.0, 152.0, 148.1, 141.2, 140.2, 131.8, 131.7, 127.2, 123.8, 120.2, 119.2, 119.1, 112.6, 110.3, 66.0, 56.0, 39.6, 38.0, 26.4, 26.2, 25.7, 25.6, 17.6, 16.7, 16.3

### Example 10: Preparation of Compound 23

### 3-(3,7-Dimethyl-octa-2,6-dienyloxy)-4-methoxy-benzoic acid methoxymethyl ester

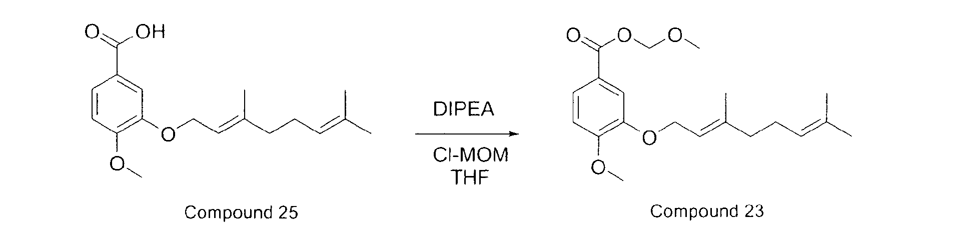

To a solution of 3-(3,7-dimethyl-octa-2,6-dienyloxy)-4-methoxy-benzoic acid [**Compound 25**, see Example 6] (172 mg, 0.56 mmol) and N,N-diisopropylethylamine (DIPEA) (83 mg, 0.67 mmol) in dry THF (2 mL) was added chloromethyl methyl ether (54 mg, 0.67 mmol) at 0°C and the resulting mixture was stirred for 12 h at room temperature. After evaporation of the solvent under reduced pressure water (100 mL) was added and the mixture was extracted with ethyl acetate (3 x 50 mL). The combined organic extracts were dried (Na₂SO₄) and the solvent removed under reduced pressure to give the title compound, (117 mg, 60%) as a brown solid.
**¹H-NMR** (CDCl₃, 400MHz, δ): 7.72 (dd, 1H, *J* = 8.4, *J* = 2.4 Hz), 7.58 (d, 1H, *J* = 2.4 Hz), 6.89 (d, 1H, *J* = 8.4 Hz), 5.53-5.46 (m, 1H), 5.46 (s, 2H), 5.09-5.04 (m, 1H), 4.67 (m, 2H), 3.92 (s, 3H), 3.53 (s, 3H), 2.15-2.02 (m, 4H), 1.76 (s, 3H), 1.64 (s, 3H), 1.58 (s, 3H).
**¹³C-NMR** (CDCl₃, 100MHz, δ ppm): 165.8, 153.7, 147.8, 141.3, 131.7, 123.7, 122.0, 119.1, 113.8, 110.3, 90.7, 65.9, 57.6, 56.0, 39.5, 26.2, 25.6, 17.6, 16.7

### Example 11: Preparation of Compound 26

### 3,4-Bis-(3,7,11-trimethyl-dodeca-2,6,10-trienyloxy)-benzoic acid ethyl ester

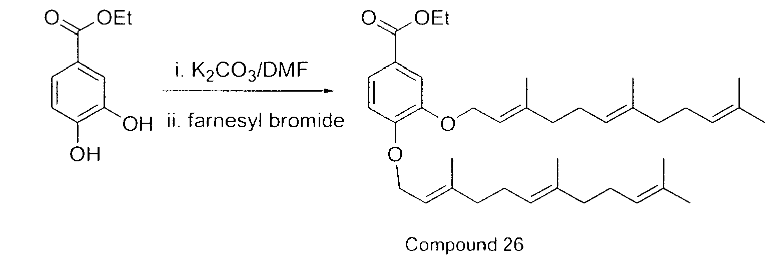

To a solution of 3,4-dihydroxy-benzoic acid ethyl ester (1.0 g, 5.5 mmol,) in DMF (20 mL), was added K₂CO₃ (1.5 g, 11.0 mmol,) and the resulting mixture was stirred for 45 minutes. Farnesyl bromide (1.8 mL 6.6 mmol,) was then added and the mixture was refluxed for 2 hours. After evaporation of the solvent under reduced pressure, water was added (100 mL) and the mixture was extracted with DCM (2 x 50 mL). The combined organic extracts were washed with saturated NaCl solution, dried (Na₂SO₄) and the solvent evaporated under reduced pressure to give **Compound 26** (335 mg, 10%).
**¹H-NMR**(CDCl₃, 400 MHz, δ ppm): 7.55 (d, 1 H, *J* = 1.7 Hz), 6.86 (d, 1H, *J* = 8.5 Hz), 5.50 (m, 2H,), 5.08 (m, 4H), 4.67 (t, 4H, *J* = 6.6 Hz), 4.34 (q, 2H, *J* = 7.1 Hz), 2.09 (m, 12H), 1.95 (m, 4H), 1.76 (s, 3H), 1.73 (s, 3H), 1.67 (s, 6H), 1.59 (s, 12H), 1.38 (t, 3H, *J* = 7.1 Hz).
¹³C-NMR (CDCl₃, 100 MHz, δ ppm): 166.49, 152.75, 148.14, 140.85, 140.78, 135.43, 135.34, 131.28, 124.34, 124.30, 123.74, 123.64, 123.37, 122.74, 119.63, 119.47, 114.44, 112.22, 66.08, 65.99, 60.66, 39.67, 39.63, 39.52, 26.71, 26.29, 26.22, 25.68, 17.67,16.77,16.01, 14.41.

### Example 12: Preparation of Compound 24

### 3,4-Bis-(3,7,11-trimethyl-dodeca-2,6,10-trienyloxy)-benzoic acid

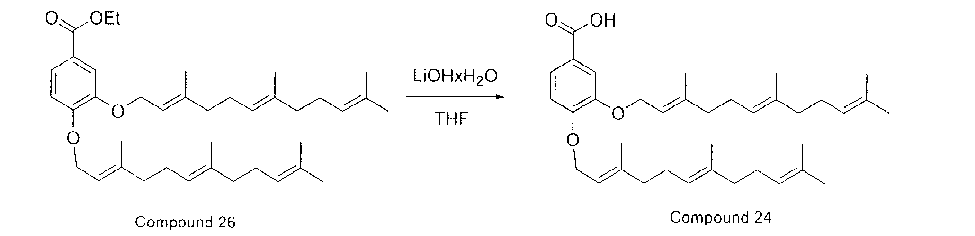

To a solution of **Compound 26,** ethyl ester derivative (142 mg, 0.24 mmol) in methanol/H₂O (3 mL, 2:1), LiOHxH₂O (160 mg, 3.84 mmol) was added and the resulting white suspension was heated to 60 °C for 4 days. After evaporation of the solvent the reaction mixture was acidified with 0.1 M HCl to pH = 3-4 and the resulting white suspension was extracted with DCM (3 x 25 mL). The combined extracts were washed with saturated NaCl solution (50 mL), dried (Na₂SO₄) and the solvent evaporated under reduced pressure to give the corresponding carboxylic acid, **Compound 24** (130 mg, 96%), as a white solid.
**¹H-NMR** (CDCl₃, 400 MHz, δ ppm): 7.72 (d, 1H, *J* = 8.6 Hz), 7.60 (s, 1H), 6.89 (d, 1H, *J* = 8.5 Hz), 5.51 (q, 2H, *J* = 6.3 Hz), 5.08 (m, 4H), 4.70 (dd, 4H, *J* = 6.2 Hz), 4.67 (dd, 4H, *J* = 6.6 Hz), 2.10 (m, 12H), 1.96 (m, 4H), 1.77 (s, 6H), 1.75 (s, 6H), 1.67 (s, 6H), 1.59 (s, 12H).
**¹³C-NMR** (CDCl₃, 100 MHz, δ ppm): 153.58, 148.18, 141.07, 140.94, 135.44, 135.35, 131.28, 131.24, 124.39, 124.34, 124.29, 123.72, 123.59, 121.37, 119.42, 119.32, 114.74, 112.16, 66.07, 66.02, 39.66, 39.61, 39.50, 26.70, 26.27, 26.18, 25.67, 17.66, 16.77, 16.75, 15.99.

### Further Spectral Data of Synthesized Compounds

Following analogous procedures to those described above, further compounds were synthesized. Their spectral data is summarized bellow:
**Compound 1**
   ¹H-NMR (CDCl₃, 400 MHz, δ ppm): 7.67 (dd, 1H, *J* = 2.0 Hz, *J* = 8.5 Hz), 7.56 (d, 1H, *J*= 2.0 Hz), 6.88 (d, 1H, *J* = 8.5 Hz), 5.52 (dt, 1H, *J* = 1.1 Hz, *J* = 6.6 Hz), 5.09 (dd, 2H, *J* = 6.9 Hz, *J* = 14.2 Hz), 4.66 (d, 2H, *J* = 6.6 Hz), 3.92 (s, 3H), 3.89 (s, 3H), 2.08-1.94 (m, 8H), 1.77 (s, 3H), 1.68 (s, 3H), 1.59 (s, 3H).
   ¹³C-NMR (25°C, CDCl₃, 100 MHz, δ ppm): 166.9, 153.4, 147.8, 141.3, 135.3, 131.2, 124.3, 123.7, 123.5, 122.5, 119.2, 113.8, 110.4, 77.3, 77.0, 76.7, 65.9, 55.9, 51.8, 39.6, 26.7, 26.2 25.6, 17.6, 16.7, 15.9
**Compound 3**
   ¹H-NMR (CDCl₃, 400 MHz, δ ppm): 6.91 (m, 4H), 5.53 (dt, 1H, *J* = 1.2 Hz, *J* = 6.4 Hz), 5.09 (dt, 1H, *J* = 1.3 Hz, *J* = 6.7 Hz), 4.62 (d, 2H, *J* = 6.5 Hz), 3.87 (s, 3H), 2.08 (m, 4H), 1.72 (s, 3H), 1.67 (s, 3H), 1.60 (s, 3H).
   ¹³C-NMR (CDCl₃, 100 MHz, δ ppm): 149.5, 148.2, 140.3, 131.7, 123.8, 120.8, 120.7, 119.9, 113.3, 111.5, 65.8, 55.8, 39.5, 26.2, 25.6, 17.6, 16.6
**Compound 4**
   ¹H-NMR (CDCl₃, 400 MHz, δ ppm): 6.90 (m, 4H), 5.53 (dt, 1H, *J* = 1.2 Hz, *J* = 6.5 Hz), 5.08 (m, 2H), 4.62 (d, 2H, *J* = 5.9 Hz), 3.87 (s, 3H), 2.06 (m, 8H), 1.73 (s, 3H), 1.68 (s, 3H), 1.60 (s, 6H).
   ¹³C-NMR (CDCl₃, 100 MHz, δ ppm): 149.5, 148.2, 140.4, 135.3, 131.2, 124.3, 123.7, 120.8, 120.6, 119.9, 113.3, 111.5, 65.8, 55.8, 39.6, 39.5, 26.7, 26.2, 25.6, 17.6, 16.6, 16.0.
**Compound 5**
   ¹H-NMR (CDCl₃, 400 MHz, δ ppm): 7.70 (m, 1H), 7.63 (m, 1H), 7.37 (t, 1H, *J* = 8 Hz), 7.16 (m, 1H), 5.52-5.44 (m, 1H), 5.12-5.06 (m, 1H), 4.58 (m, 2H), 3.90 (m, 3H), 2.18-2.06 (m, 4H), 1.74 (s, 3H), 1.66 (s, 3H), 1.60 (s, 3H).
   ¹³C-NMR (CDCl₃, 100 MHz, δ ppm): 167.0, 156.0, 141.6, 131.5, 129.6, 129.4, 123.7, 122.0, 120.2, 116.2, 114.7, 65.0, 52.2, 39.5, 26.2, 25.6, 17.6, 16.6.
**Compound 6**
   ¹H-NMR (CDCl₃, 400 MHz, δ ppm): 7.40 (m, 2H), 7.20 (m, 1H), 7.08 (m, 1H), 5.42-5.38 (m, 1H), 5.09-5.06 (m, 1H), 4.45 (m, 2H), 2.18-2.06 (m, 4H), 1.74 (s, 3H), 1.66 (s, 3H), 1.60 (s, 3H).
   ¹³C-NMR (CDCl₃, 100 MHz, δ ppm):172.0, 158.8, 138.7, 130.4, 129.3, 122.5, 121.2, 119.2, 115.2, 114.1, 110.3, 65.0, 40.0, 26.5, 25.8, 18.2, 17.1.
**Compound 7**
   ¹H-NMR(CDCl₃, 400 MHz, δ ppm): 7.70 (m, 1H), 7.63 (m, 1H), 7.37 (t, 1H, *J*=8), 7.16 (m, 1H), 5.52-5.48 (m, 1H), 4.57 (m, 2H), 1.81 (s, 3H), 1.77 (s, 3H).
   ¹³C-NMR(CDCl₃, 100 MHz, δ ppm): 172.0, 158.8, 138.7, 130.4, 129.4, 122.5, 121.2, 119.2, 115.1, 65.0, 25.8, 18.2.
**Compound 10**
   ¹H-NMR (CDCl₃, 100 MHz, δ ppm): (7.65, d, 1H, *J* = 8.4 Hz), (6.77, d, 1H, *J* = 8.8 Hz), (5.58-5.54, m, 1H), (5.15-5.03, m, 3H), (4.45, d, 2H, *J* = 6.8 Hz), (3.88, s, 3H), (3.82, s, 3H), (3.77, d, 2H, *J* = 6.4 Hz), 2.11-1.92, m, 8H), (1.74, s, 3H), (1.70,s, 6H), (1.63, s, 3H), (1.60, s, 3H), (1.55, s, 3H)
   ¹³C-NMR (CDCl₃): 156.1, 146.5, 141.2, 139.0, 135.2, 131.3, 127.5, 124.6, 124.2, 123.7, 123.2, 120.5, 109.0, 70.0, 56.0, 52.0, 40.0, 39.0, 27.0, 26.6, 26.2, 26.0, 25.8, 18.0, 16.6
**Compound 13**
   ¹H-NMR (CDCl₃, 400 MHz, δ ppm): 7.66 (dd, 1H, *J* = 8.4 Hz, *J* = 2 Hz), 7.55 (d, 1H, *J* = 2 Hz), 6.86 (d, 1H, *J* = 8.4 Hz), 5.52-5.44 (m, 2H), 5.09-5.05 (m, 1H), 4.78 (m, 2H), 4.66 (2H, m), 3.91 (s, 3H), 2.12-2.04 (m, 4H), 1.78 (s, 3H), 1.76 (s, 3H), 1.65 (s, 3H), 1.58 (s, 3H), 1.57 (s, 3H).
**Compound 14**
   ¹H-NMR (CDCl₃, 400 MHz, δ ppm):7.66 (dd, 1H, *J* = 8.4 Hz, *J* = 2 Hz), 7.55 (d, 1H, *J* = 2 Hz), 6.86 (d, 1H, *J* = 8.4Hz), 5.52-5.44 (m, 2H), 5.13-5.06 (m, 3H), 4.81 (m, 2H), 4.66 (2H, m), 3.92 (s, 3H), 2.13-2.04 (m, 12H), 1.77 (s, 6H), 1.68 (s, 3H), 1.66 (s, 3H), 1.60 (s, 3H), 1.59 (s, 6H).
   ¹³C-NMR (CDCl₃, 100 MHz, δ ppm):166.4, 153.2, 147.7, 142.0, 141.2, 135.4, 131.7, 131.2, 124.6, 124.0, 123.6, 123.4, 123.0, 119.3, 118.6, 113.7, 110.36, 65.8, 62.0, 56.0, 39.6, 39.6, 39.5, 26.7, 26.2, 26.2, 25.7, 25.6, 17.6, 16.6, 16.5, 16.0.
**Compound 15**
   ¹H NMR (400 MHz, CDCl₃):
      7.52, 7.45, 6.62, 5.46, 4.54, 4.31, 3.83, 1.76, 1.72
   ¹³C NMR (100MHz, CDCl₃):
      167.10, 145.08, 141.46, 137.90, 123.84, 119.39, 118.90, 112.87, 112.22, 65.07, 51.41, 25.56, 17.97
**Compound 16**
   ¹H NMR (400 MHz, CDCl₃):
      7.63, 7.52, 6.68, 5.50, 4.60, 1.80, 1.76
   ¹³C NMR (100MHz, CDCl₃):
      172.54, 145.17, 142.26, 138.27, 124.99, 119.41, 118.26, 113.02, 112.79, 65.27, 25.75, 18.18
**Compound 18**
   ¹H NMR (400 MHz, CDCl₃):
      8.41, 7.95, 7.62, 7.50, 5.44, 4.59, 3.86, 2.19, 1.78, 1.74
   ¹³C NMR (100MHz, CDCl₃):
      168.33, 166.63, 146.20, 138.95, 132.13, 124.63, 123.06, 118.67, 118.40, 111.78, 65.66, 51.89, 25.69, 24.91, 18.14
**Compound 20**
   ¹H NMR (400 MHz, CDCl₃):
      8.42, 7.95, 7.62, 7.51, 5.44, 5.06, 4.62, 3.85, 2.18, 2.06, 1.99, 1.74, 1.64, 1.57, 1.56
   ¹³C NMR (100MHz, CDCl₃):
      168.21, 166.56, 146.15, 142.13, 135.37, 132.11, 131.13, 124.60, 124.10, 123.34, 123.05, 118.48, 118.36, 111.82, 65.69, 51.82, 39.53, 39.42, 26.54, 26.03, 25.52, 24.85, 17.52, 16.57, 15.86
**Compound 22**
   ¹H-NMR (CDCl₃, 400 MHz, δ ppm): 7.63 (d, 1H, *J* = 8.3 Hz), 7.56 (s, 1H), 6.93 (d, 1H, *J* = 8.3 Hz), 6.08 (d, 1H, *J* = 2.1 Hz), 5.48 (t, 1H, *J* = 6.6 Hz), 5.10 (d, 2H, *J* = 5.7 Hz), 4.66 (d, 2H, *J* = 6.7 Hz), 4.35 (q, 2H, *J* = 7.2 Hz), 2.17-1.97 (m, 8H), 1.76 (s, 3H), 1.68 (s, 3H), 1.60 (s, 6H), 1.38 (t, 3H, *J* = 7.1 Hz).
   ¹³C-NMR (CDCl₃, 100 MHz, δ ppm): 166.42, 150.20, 145.35, 142.63, 135.57, 131.34, 124.25, 124.00, 123.49, 122.48, 118.56, 113.91, 113.02, 65.97, 60.71, 39.66, 39.55, 26.68, 26.18, 25.67, 17.67, 16.70, 16.01, 14.39.

### Biological Methods

### BACE ASSAY

The aim of this assay is to determine if a compound, either synthetic or of marine origin, is a BACE-1 inhibitor, to avoid the formation of Aβ. This assay is based on FRET technology (Fluorescence Resonance Energy Transfer). FRET is used to measure cleavage of a peptide substrate, among other uses. The peptide substrate shows two fluorophores, a fluorescence donor and a quenching acceptor. The distance between these two fluorophores has been selected so that upon light excitation, the donor fluorescence energy is significantly quenched by the acceptor. When a substrate peptide cleavage occurs, the energy balance is broken and all the donor fluorescence can be observed. The increase in fluorescence is linearly related to the rate of proteolysis (Gordon, GW et al., 1998). In this assay the reaction occurs between an enzyme, purified BACE-1, and a fluorogenic peptidic substrate who present the "Swedish mutation". The peptide cleavage by BACE-1 produces fluorescence energy and enzymatic activity can be quantified.

The reagents which are used in this assay are the following:
- rhBACE-1 β-Secretase recombinant human (R&D Systems. Ref. 931-AS).
- Fluorogenic Peptide Substrate IV (R&D Systems. Ref. ES004).
- Beta-SECRETASE INHIBITOR H-4848. (BACHEM. Ref. H-4848.0001).
- Sodium acetate.

The assay is carried out in a 96 wells microplate. The final concentration of substrate is 3,5 µM per well, and the enzyme concentration is 0,5 µg/ml. The final volume of the assay is 100 µl per well and all reagents are diluted in Reaction Buffer. The compounds are tested at a concentration of 10⁻⁵ and 10⁻⁶ M. The control in the assay is the commercial inhibitor β-Secretase inhibitor H-4848 from BACHEM, which is tested at 300 nM. All the samples and controls are studied by duplicate.

The plate is mixed gently and changes in the fluorescence are measured using a fluorimeter plate reader, with 320 nm excitation filter and 405 nm emission filter. The temperature should be preferably maintained between 25 and 30 °C. Measurements are carried out every ten minutes during an hour. The first measure is subtracted from the last to calculate the fluorescence increase, evaluating the enzymatic activity. The 100% activity is calculated as the mean of the results of wells without sample or inhibitor.

In the cases where abnormal effects in fluorescence were detected, BACE inhibition activity was assayed using BACE-1 (beta-Secretase) FRET ASSAY KIT (Invitrogen, Ref. P2985). Fluorescence was measured with a fluorimeter plate reader, with 544 nm excitation filter and 580 nm emission filter.

Further information regarding this assay may be found in the following references, which are incorporated by reference into the present application:
Andrau, D et al; "BACE1- and BACE2-expressing human cells: characterization of beta-Amyloid precursor protein-derived catabolites, design of a novel fluorimetric assay, and identification of new in vitro inhibitors". J Biol Chem. 2003 Jul 11;278(28):25859-66.
Gordon, GW et al; "Quantitative fluorescence resonance energy transfer measurements using fluorescence microscopy." Biophys J. 1998 May; 74:2702-13.

### GSK-3 beta INHIBITION ASSAY

The GSK-3 beta activity of the compounds of formula (I) according to the present invention was determined by incubation of a mixture of recombinant human GSK-3 enzyme, a phosphate source and GSK-3 substrate in the presence and in the absence of the corresponding test compound, and by measuring the GSK-3 activity of this mixture. The compounds where tested at final concentrations of 25 and 50 µM.

Recombinant human glycogen synthase kinase 3 beta was assayed in MOPS 11 mM, pH 7.4, EDTA 0.2 mM, EGTA 1.25 mM, MgCl₂ 26.25 mM and sodium orthovanadate 0.25 mM in the presence of 62.5 µM of Phospho-Glycogen Synthase Peptide-2 (GS-2), 0.5 µCi gamma-³³P-ATP and unlabelled ATP at a final concentration of 12.5 µM. The final assay volume was 20 µl. After incubation for 30 minutes at 30 °C, 15 µl aliquots were spotted onto P81 phosphocellulose papers. Filters were washed four times for at least 10 minutes each and counted with 1.5 ml of scintillation cocktail in a scintillation counter.

The compounds of formula (I) of the present invention where submitted to the above indicated assays, in order to determine both their GSK-3 inhibition activity and BACE activity inhibition. The results are indicated in Table I, in percentage of the respective enzyme activity.

| | **% GSK-3 beta activity** | | **% of BACE activity** | |
|---|---|---|---|---|
| | **25 µM** | **50 µM** | **1 µM** | **10 µM** |
| Compound 2 | 88.06 | 67.98 | 100 | 18.5 ± 2.1 |
| Compound 5 | 94.37 | 80.7 | 13.3 | 0 |
| Compound 6 | 70.28 | 79.95 | 87.5 ± 17 | 36.5 ± 38 |
| Compound 8 | 85.57 | 71.01 | 100 | 81 ± 8 |
| Compound 9 | 91.7 | 59.47 | 100 | 80 ± 13 |
| Compound 10 | 55.99 | 19.48 | 86.5 ± 5 | 82 ± 10 |
| Compound 11 | 84.77 | 58.19 | 48 ± 2 | 8 ± 3 |
| Compound 12 | 47.48 | 27.91 | 99 ± 7 | 42 ± 18 |
| Compound 13 | 54.56 | 7.36 | 49 ± 5 | 0 |
| Compound 17 | 90.42 | 65.51 | 100 | 79 ± 13 |
| Compound 19 | / | 85.11 | 3.5 ± 5 | 0 |
| Compound 20 | 74.41 | 39.99 | 2 ± 4.5 | 0 |
| Compound 21 | 90 | 57.5 | 68.5 ± 0.7 | 29 ± 10 |
| Compound 23 | 53 | 18.6 | 61 ± 33 | 0 |
| Compound 24 | 12.45 | 4.55 | 30± 7 | 26 ± 13 |

## Claims

1. A compound of formula (I) 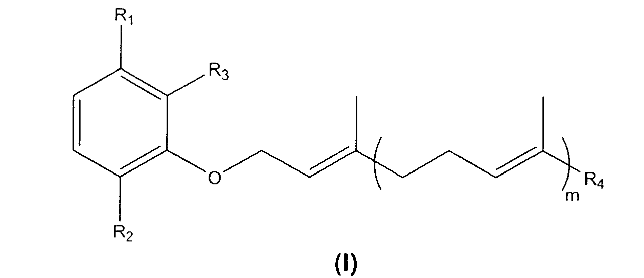 wherein
m is an integer selected from 0, 1, 2, 3, 4, 5 or 6;
R₁ is selected from hydrogen, -C(=O)OCH₂OCH₃, -(CH₂)ᵣC(=O)OR₅ and -(CH₂)ᵣC(=O)NHR₅, wherein
r is an integer selected from 0, 1, 2, 3 or 4; and
R₅ being selected from hydrogen, C₁-C₁₂ alkyl, C₂-C₁₂ alkenyl, C₂-C₁₂ alkynyl, and 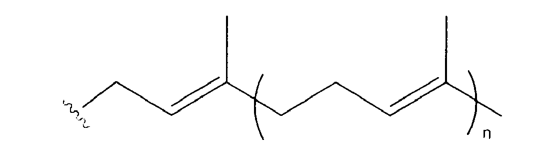 wherein n is an integer selected from 0, 1, 2, 3, 4, 5 or 6;
R₂ is selected from hydrogen, -NH₂, -NHC(=O)R₆ and -OR₆,
R₆ being selected from hydrogen, C₁-C₆ alkyl and 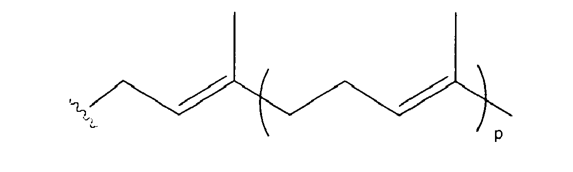 wherein p is an integer selected from 0, 1, 2, 3, 4, 5 or 6;
R₃ is selected from hydrogen and 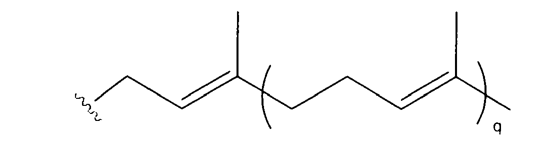 wherein q is an integer selected from 0, 1, 2, 3, 4, 5 or 6;
and R₄ is selected from -CH₃, -CH₂-CH₃, -CH₂-OH and -CH₂-CH₂-OH;
with the proviso that at least one of R₁ and R₂ is not hydrogen, and the provisos that:
when m is 0, R₂ and R₃ are hydrogen and R₄ is -CH₃, R₁ may not be -CH₂-C(=O)O-CH₂-CH₃;
when m is 0, R₁ and R₃ are hydrogen and R₄ is -CH₃, R₂ may not be -OH nor-OCH₃;
when m is 1, R₁ and R₃ are hydrogen and R₄ is -CH₃, R₂ may not be -OH nor-OCH₃;
when m is 2, R₁ and R₃ are hydrogen and R₄ is -CH₃, R₂ may not be -OH;
when m is 0, R₁ and R₃ are hydrogen, R₄ is -CH₃, R₂ may not be -O-CH₂-CH=C(CH₃)₂,
when m is 0, R₁ and R₃ are hydrogen, R₄ is -CH₃, R₂ may not be -NH₂;
and any pharmaceutically acceptable salts, solvates and prodrugs thereof.

2. Compound according to claim 1, wherein R₁ is selected from hydrogen, -C(=O)OH, -C(=O)OCH₃, -C(=O)O-CH₂-CH₃, -C(=O)NHR₅ and C(=O)OR₅, wherein R₅ is 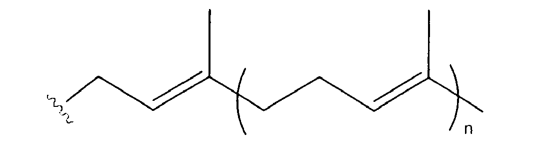 wherein n is an integer selected from 0, 1, 2 or 3.

3. Compound according to any of the previous claims, wherein R₂ is selected from hydrogen, -OH, -OCH₃, -NH₂, -NHC(=O)CH₃, -OR₆, wherein R₆ is 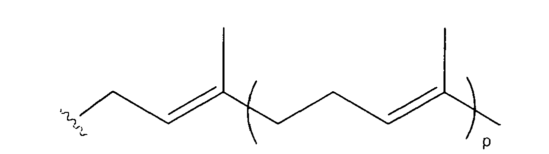 wherein p is an integer selected from 0, 1, 2 or 3.

4. Compound according to any of the previous claims, wherein R₃ is hydrogen.

5. Compound according to any of the previous claims, wherein R₄ is -CH₃.

6. Compound according to any of the previous claims, wherein m is an integer selected from 0, 1, 2 or 3.

7. Compound according to claim 1, wherein the compound of formula (I) is selected from 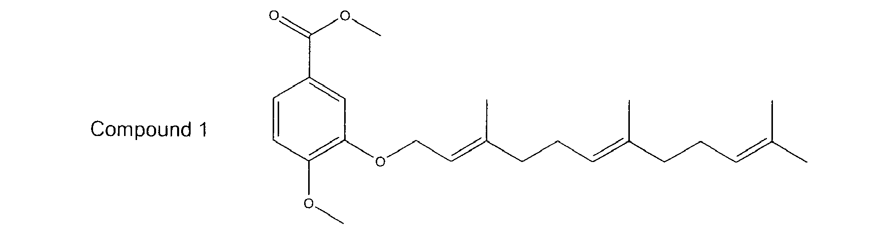 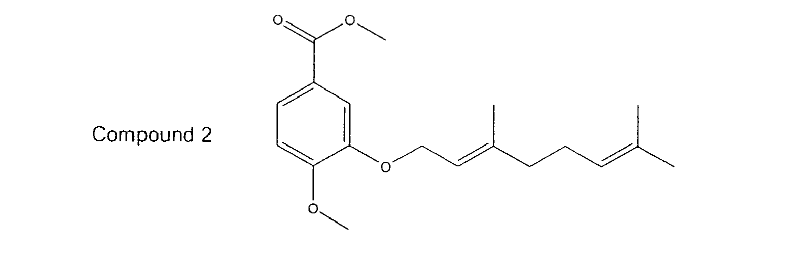 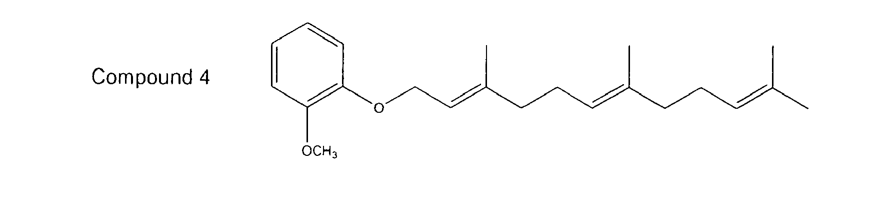 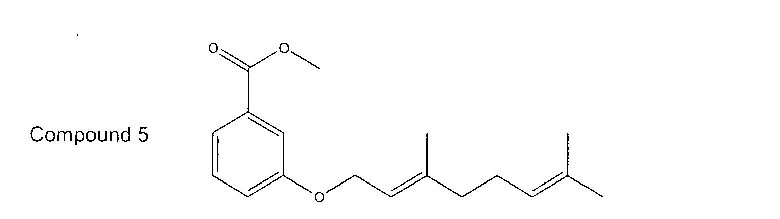 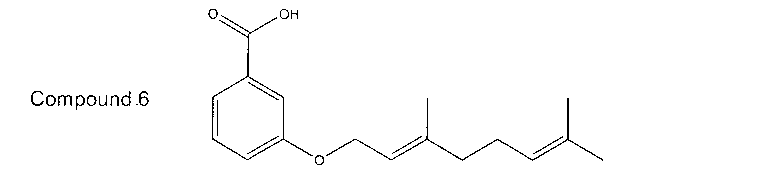 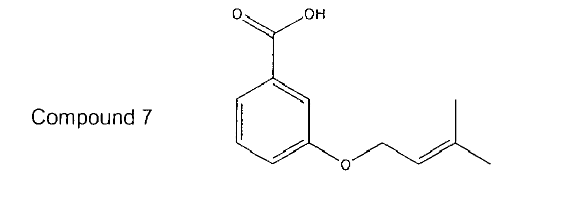 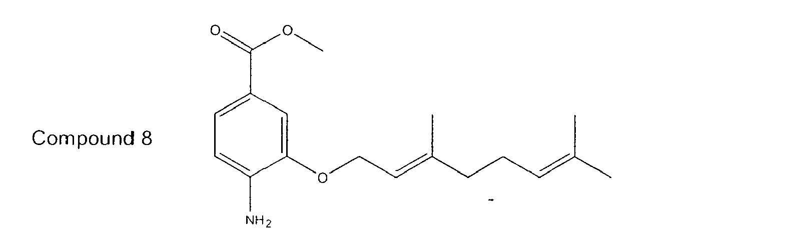 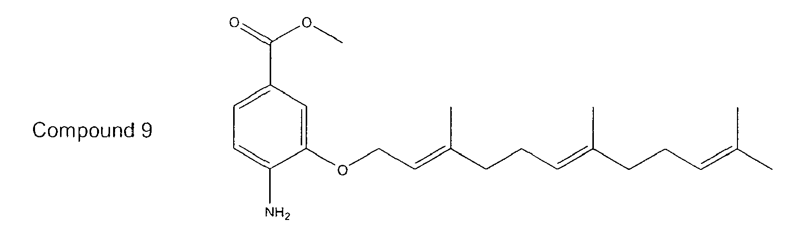 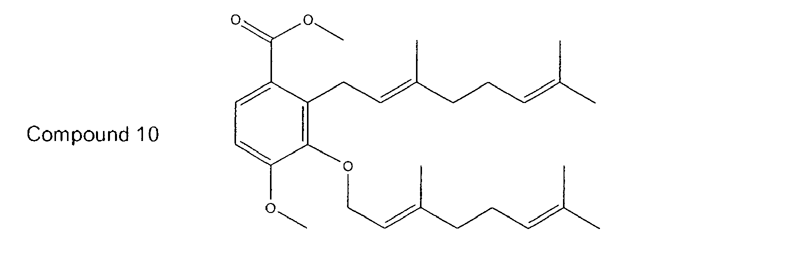 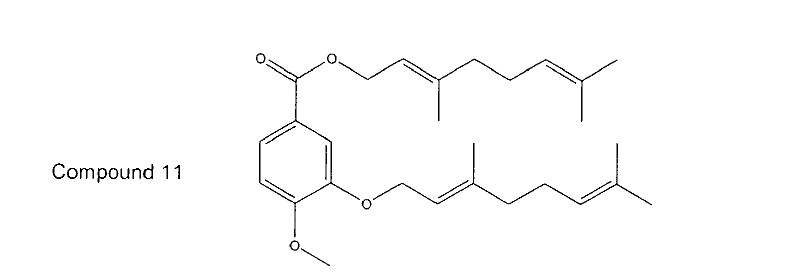 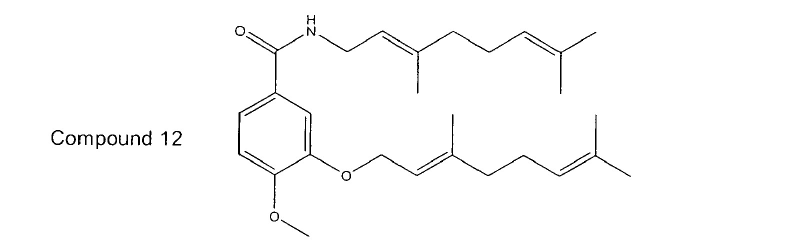 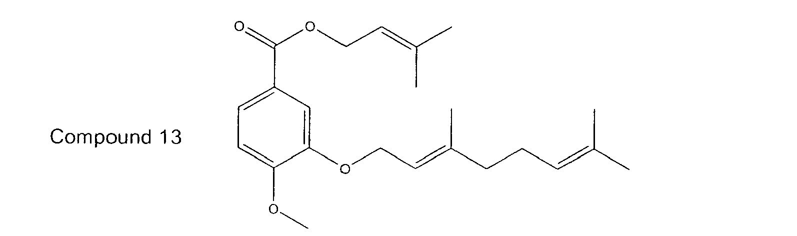 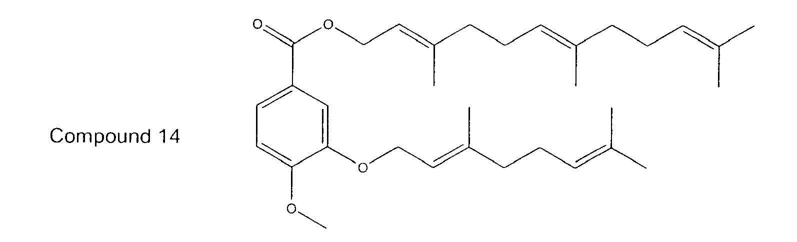 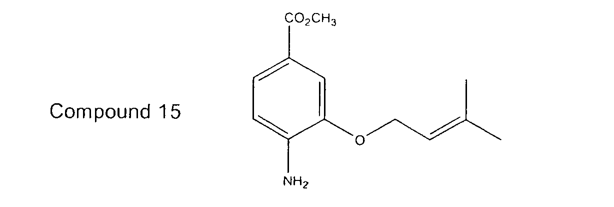 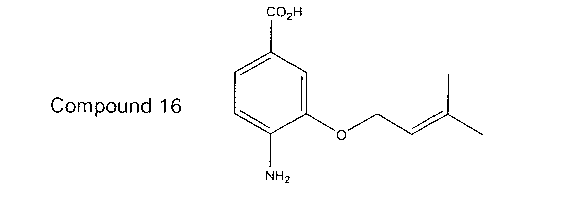 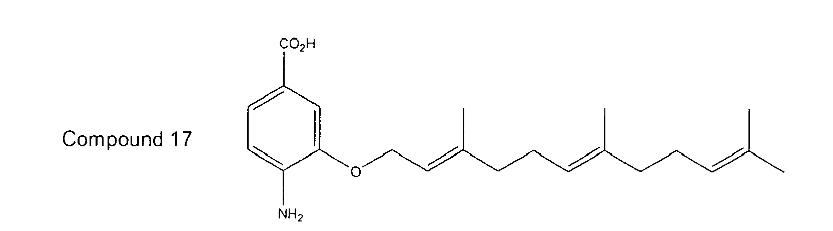 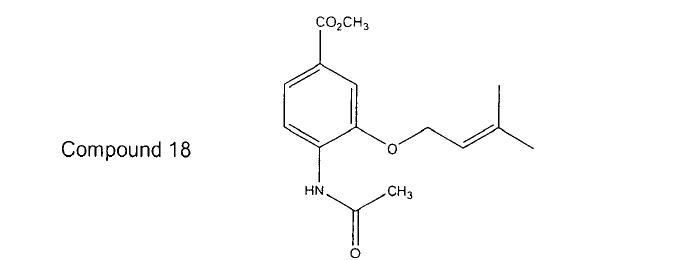 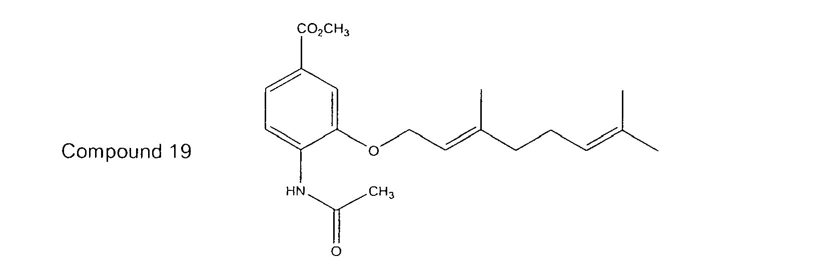 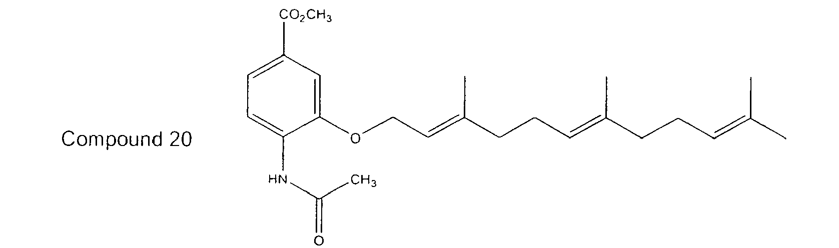 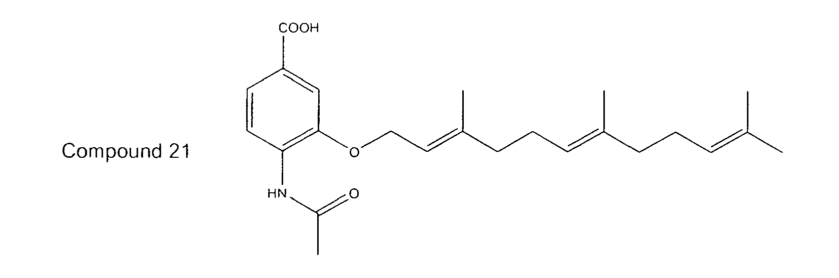 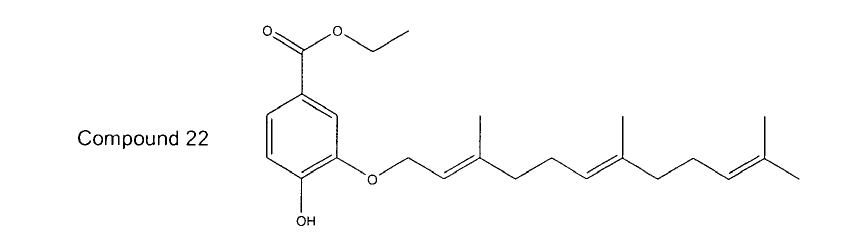 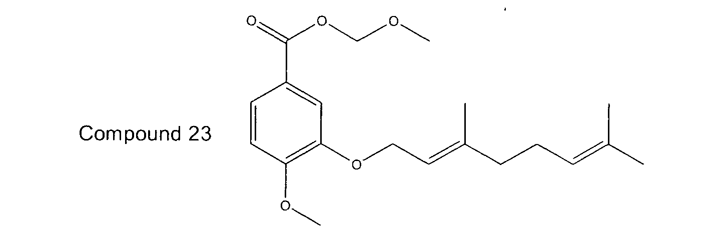 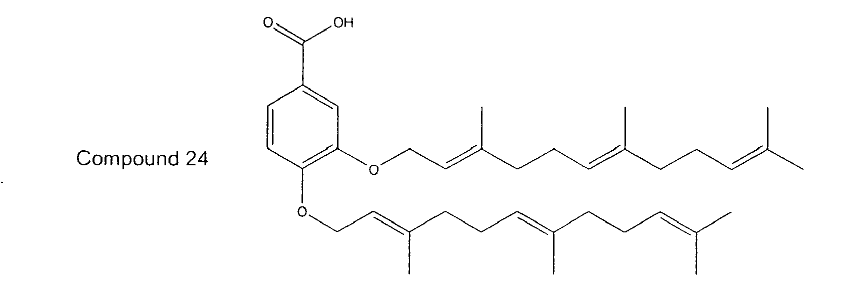 and any pharmaceutically acceptable salts, solvates and prodrugs thereof.

8. A compound of formula (I) as defined in any one of claims 1 to 7 for use as a medicament.

9. A pharmaceutical composition comprising at least one of the compounds of formula (I) as defined in any one of claims 1 to 7, or salts, prodrugs or solvates thereof, together with at least one pharmaceutically acceptable carrier, adjuvant and/or vehicle.

10. Use of a compound of formula (I) 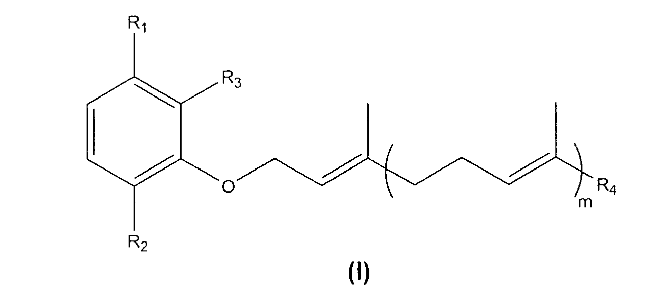 wherein
m is an integer selected from 0, 1, 2, 3, 4, 5 or 6;
R₁ is selected from hydrogen, -C(=O)OCH₂OCH₃, -(CH₂)ᵣC(=O)OR₅ and -(CH₂)ᵣC(=O)NHR₅, wherein
r is an integer selected from 0, 1, 2, 3 or 4;and
R₅ being selected from hydrogen, C₁-C₁₂ alkyl, C₂-C₁₂ alkenyl, C₂-C₁₂ alkynyl, and 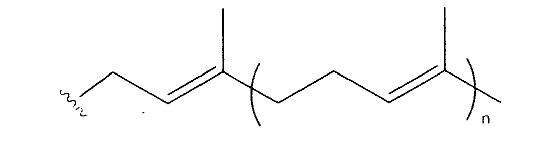 wherein n is an integer selected from 0, 1, 2, 3, 4, 5 or 6;
R₂ is selected from hydrogen, -NH₂, -NHC(=O)R₆ and -OR₆,
R₆ being selected from hydrogen, C₁-C₆ alkyl and 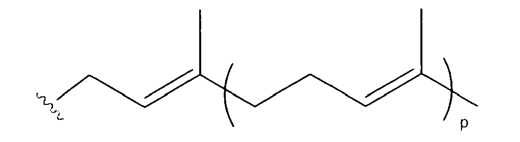 wherein p is an integer selected from 0, 1, 2, 3, 4, 5 or 6;
R₃ is selected from hydrogen and 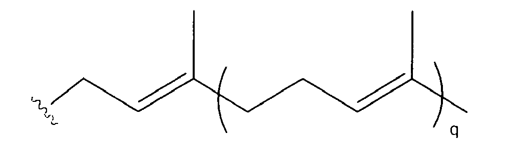 wherein q is an integer selected from 0, 1, 2, 3, 4, 5 or 6;
and R₄ is selected from -CH₃, -CH₂-CH₃, -CH₂-OH and -CH₂-CH₂-OH;
and any pharmaceutically acceptable salts, solvates and prodrugs thereof;
in the manufacture of a medicament for the treatment and/or profilaxis of a cognitive, neurodegenerative or neuronal disease or disorder.

11. Use according to claim 10, wherein the disease or disorder is selected from conditions associated with chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding, such as cerebral bleeding due to solitary cerebral amyloid angiopathy, mild cognitive impairment, Hereditary Cerebral Hemmorhage with Amyloidosis of the Dutch-Type, cerebral Amyloid angiophathy, ischaemia, brain injury, especially traumatic brain injury, Down's syndrome, Lewy body disease, inflammation and chronic inflammatory diseases

12. Use according to claim 11, wherein the disease or disorder is selected from chronic neurodegenerative conditions including dementias such as Alzheimer's disease and Parkinson's disease, Huntington's Disease, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding, mild cognitive impairment, ischaemia, brain injury, especially traumatic brain injury, inflammation and chronic inflammatory diseases.

13. Use according to claim 12, wherein the disease or disorder is selected from Alzheimer's Disease, Parkinson's Disease, multiple sclerosis, stroke, epilepsy, mood disorders, ischaemia, brain injury and chronic inflammatory diseases.

14. A process for the preparation of a compound of formula (I) as defined in any one of claims 1 to 7, comprising reacting the corresponding phenol of formula (A) 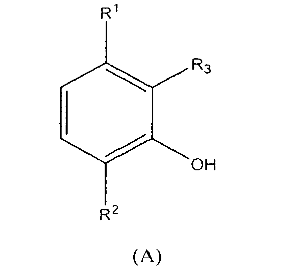 wherein R₁, R₂ and R₃ are as defined in claim 1;
with a suitable unsaturated alkyl bromide of formula (B) 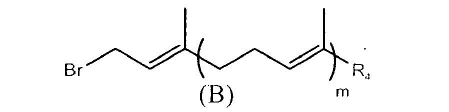 wherein m is as defined in claim 1;
in the presence of a base.

15. Method of treating and/or preventing a cognitive, neurodegenerative or neuronal disease or disorder, which method comprises administering to a patient in need of such a treatment a therapeutically effective amount of at least one compound of formula (I) as defined in any of claims 1 to 7 or a pharmaceutical composition thereof.

16. Method according to claim 15, the disease or disorder being selected from chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding, such as cerebral bleeding due to solitary cerebral amyloid angiopathy, mild cognitive impairment, Hereditary Cerebral Hemmorhage with Amyloidosis of the Dutch-Type, cerebral Amyloid angiophathy, ischaemia, brain injury, especially traumatic brain injury, Down's syndrome, Lewy body disease, inflammation and chronic inflammatory diseases.
